(19) Europäisches Patentamt
European Patent Office
Office européen des brevets

(11) Veröffentlichungsnummer: **0 076 922**
**B1**

(12) # EUROPÄISCHE PATENTSCHRIFT

(45) Veröffentlichungstag der Patentschrift: **19.06.85**

(21) Anmeldenummer: **82108084.3**

(22) Anmeldetag: **02.09.82**

(51) Int. Cl.⁴: **C 09 B 62/513**, C 09 B 62/09, D 06 P 3/66, D 06 P 3/10, C 07 D 251/50

(54) **Wasserlösliche Disazoverbindungen und deren neue Kupplungskomponenten, Verfahren zu ihrer Herstellung und Verwendung der Disazoverbindungen als Farbstoffe.**

(30) Priorität: **08.09.81 DE 3135432**

(43) Veröffentlichungstag der Anmeldung: **20.04.83 Patentblatt 83/16**

(45) Bekanntmachung des Hinweises auf die Patenterteilung: **19.06.85 Patentblatt 85/25**

(84) Benannte Vertragsstaaten: **CH DE FR GB IT LI**

(56) Entgegenhaltungen:
**EP - A - 0 014 432**
**GB - A - 2 008 144**

(73) Patentinhaber: **HOECHST AKTIENGESELLSCHAFT, Postfach 80 03 20, D-6230 Frankfurt am Main 80 (DE)**

(72) Erfinder: **Fuchs, Hermann, Dr., Altenhainer Strasse 2, D-6240 Königstein/Taunus (DE)**

**EP 0 076 922 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung liegt auf dem technischen Gebiet der faserreaktiven Disazofarbstoffe.

Die Europäische Patentanmeldungs-Veröffentlichung Nr. 0 014 432 A2 beschreibt Disazofarbstoffe, die aus einer Bis-(aminophenoxy)-methan- oder Bis-(amino-nitrophenoxy)-methan-Verbindung als Tetrazokomponente und aus zwei Äquivalenten einer wasserlöslichen, eine faserreaktive Gruppe enthaltenden Kupplungskomponente aufgebaut sind.

Aus der britischen Patentanmeldungs-Veröffentlichung Nr. 2 008 144 A sind aus den Beispielen 222 bis 229 Disazofarbstoffe bekannt, die aus zwei Monoazokomponenten bestehen, die über die Aminogruppen der 1-Amino-8-hydroxynaphthalin-3,6-disulfonsäure als deren Kupplungskomponente und jeweils einem Chlortriazinrest mit den Aminogruppen des $\alpha,\beta$-Bis-(4-aminophenoxy)-äthans als Brückenglied miteinander verbunden sind. Die Diazokomponenten der Monoazokomponenten enthalten die faserreaktive $\beta$-Sulfatoäthylsulfonyl-Gruppe.

Mit der vorliegenden Erfindung wurden neue wasserlösliche, symmetrische Disazoverbindungen der allgemeinen Formel (1)

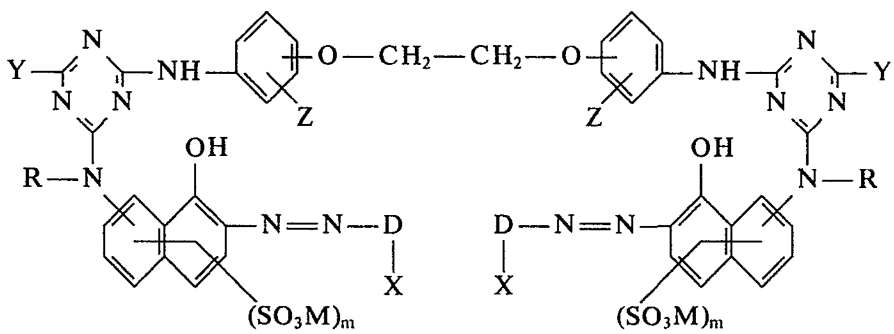

(1)

gefunden, in welcher die zweifach auftretenden Formelglieder D, R, X, Y, Z, M und m jeweils eine einander identische Bedeutung besitzen und die Gruppen Z an die Benzolkerne jeweils in ortho- oder jeweils in para-Stellung zum Äthylendioxy-Substituenten sowie die Aminogruppen —NH— und die Gruppen Z in den Benzolkernen jeweils zueinander meta-ständig gebunden sind und

D der Rest einer aromatischen Diazokomponente ist,
X ein Wasserstoffatom oder eine faserreaktive Gruppe bedeutet, wobei X auch die Bedeutung des nachstehend definierten Formelrestes Z haben kann,
Y ein Fluoratom oder ein Chloratom ist,
Z eine Gruppe der Formel

$$-SO_2-CH_2{=}CH_2 \text{ oder } -SO_2-CH_2-CH_2-R'$$

bedeutet, in welcher
R' einen anorganischen oder organischen, im wäßrigen Medium alkalisch eliminierbaren Rest darstellt oder für die Hydroxygruppe steht,
M ein Wasserstoffatom oder ein Alkalimetall, wie Lithium und insbesondere Natrium oder Kalium, oder das Äquivalent eines Erdalkalimetalls, wie des Calciums, ist,
R ein Wasserstoffatom oder eine Alkylgruppe von 1 bis 4 C-Atomen bedeutet und
m die Zahl 1 oder 2 ist.

Sofern X nicht die faserreaktive Gruppe Z selbst ist, ist der Rest X an den Rest D über eine Aminogruppe der Formel —NR''—, in welcher R'' ein Wasserstoffatom oder eine Alkylgruppe von 1 bis 4 C-Atomen bedeutet, verknüpft.

Anorganische oder organische Reste R' der faserreaktiven Gruppe $-SO_2-CH_2-CH_2-R'$ sind beispielsweise eine Alkansulfonyloxy-Gruppe von 1 bis 4 C-Atomen, wie die Methansulfonyloxygruppe, eine Dialkylaminogruppe mit Alkylgruppen von jeweils 1 bis 4 C-Atomen, wobei die Alkylgruppen zueinander gleich oder voneinander verschieden sein können und jede eine Carboxygruppe (der allgemeinen Formel —COOM mit M der obengenannten Bedeutung) oder nur eine der Alkylgruppen eine Carboxygruppe oder eine Sulfogruppe (der allgemeinen Formel $-SO_3M$ mit M der obengenannten Bedeutung) enthalen kann, wie z. B. die Diäthylamino-, die N-Methyl-N-$\beta$-sulfoäthylamino- oder die Amino-diessigsäure-Gruppe, und bevorzugt ein Halogenatom, wie das Fluor-, Chlor- oder Bromatom, eine Thiosulfatogruppe (entsprechend der allgemeinen Formel $-S-SO_3M$ mit M der oben angegebenen Bedeutung), eine Phosphatogruppe (entsprechend der allgemeinen Formel $-O-PO_3M_2$ mit M der oben angegebenen Bedeutung), die Amidosulfonyloxygruppe der Formel $-O-SO_2NH_2$ und insbesondere bevorzugt eine Sulfatogruppe (entsprechend

allgemeinen Formel $-OSO_3M$ mit M der oben angegebenen Bedeutung).

Das Formelglied D ist bevorzugt ein Phenylenrest oder ein Naphthylenrest, das außer dem Formelglied X und der Azogruppe noch andere für Azofarbstoffe übliche Substituenten enthalten kann; als solche Substituenten sind insbesondere zu nennen: Halogenatome, wie das Chlor- und Bromatom, Sulfogruppen, Sulfamoylgruppen, die durch niedere Alkyl-, Phenyl- und/oder niedere Phenylalkyl-Reste mono- oder disubstituiert sein können, niedere Alkylgruppen, niedere Alkoxygruppen, Aryloxygruppen, Carboxygruppen, Carbamoylgruppen, die durch niedere Alkyl-, Phenyl- und/oder niedere Phenylalkyl-Reste mono- oder disubstituiert sein können, niedere Alkyloxycarbonyl-Gruppen und Acylaminogruppen, in welchen der Acylrest eine niedere Alkanoylgruppe, eine niedere Arylalkanoylgruppe oder eine gegebenenfalls durch niedere Alkylgruppen substituierte Aroylgruppe sein kann. Bevorzugt ist D ein Phenylenrest, der durch einen oder zwei Substituenten aus der Gruppe Chlor, Brom, Sulfo, niederes Alkyl, niederes Alkoxy und Carboxy substituiert sein kann. Ebenso ist D bevorzugt ein Naphthylenrest, der durch eine, zwei oder drei Sulfogruppen substituiert ist.

Der hier verwendete Ausdruck »niedere(s)« bedeutet im vorstehenden als auch im nachstehenden Gebrauch, daß die hiermit bezeichnete Gruppe oder der hiermit bezeichnete Rest aus einer Alkylgruppe von 1 bis 4 C-Atomen besteht oder einen Alkylrest oder Alkylenrest von 1 bis 4 C-Atomen enthält. Niedere Alkylgruppen, die durch einen Arylrest substituiert sind, sind bevorzugt die Phenäthyl- und insbesondere die Benzylgruppe.

Die vorstehend und auch nachstehend genannten Arylreste sind bevorzugt die Phenylgruppe oder eine Naphthylgruppe, wobei die Phenylgruppe noch durch einen oder zwei Substituenten aus der Gruppe Methoxy, Äthoxy, Methyl, Äthyl, Chlor, Carboxy und Sulfo substituiert sein kann und die Naphthylgruppe noch durch eine Methyl-, Äthyl-, Methoxy-, Äthoxy- oder Carboxygruppe oder ein Chloratom und/oder eine, zwei oder drei Sulfogruppen substituiert sein kann.

Faserreaktive Gruppen X, die in den erfindungsgemäßen Disazoverbindungen der allgemeinen Formel (1) enthalten sein können, sind die zahlreich in der Literatur bekannten faserreaktiven Reste der carbocyclischen und heterocyclischen sowie aliphatischen Reihe, bevorzugt jedoch der 2,3-Dichlorchinoxalincarbonyl-Rest, der 2,4-Difluor-5-chlor-pyrimidyl-Rest, der 2,4-Dichlor-s-triazinyl-Rest, der 2-Chlor-4-(niederes Alkoxy)-s-triazinyl-Rest und der 2-Fluor- sowie der 2-Chlor-4-amino-s-triazinyl-Rest, in welchen die Aminogruppe durch niedere Alkyl-, Aryl- und/oder niedere Arylalkyl-Reste substituiert sein kann, und ebenso die oben genannte Gruppe Z, wobei diese faserreaktiven Gruppen über die obengenannte Gruppe $-NR''-$ an D gebunden ist, oder X ist, wie bereits erwähnt, die faserreaktive Gruppe Z selbst.

Die aus der allgemeinen Formel (1) ersichtlichen Sulfoaminonaphtol-Komponenten der erfindungsgemäßen Verbindungen leiten sich bevorzugt von folgenden Aminonaphtholsulfonsäuren ab:

1-Amino-8-naphthol-3,6-disulfonsäure, 1-Amino-8-naphthol-4,6-disulfonsäure,
2-Amino-8-naphthol-6-sulfonsäure, 2-Methylamino-8-naphthol-6-sulfonsäure,
2-Amino-5-naphthol-7-sulfonsäure, 2-Methylamino-5-naphthol-7-sulfonsäure,
2-Amino-8-naphthol-3,6-disulfonsäure, 2-Amino-5-naphthol-1,7-disulfonsäure,
2-Amino-8-naphthol-5-sulfonsäure.

Die neuen Disazoverbindungen der allgemeinen Formel (1) können sowohl in saurer Form als auch in Form ihrer Salze, wie der Salze der Sulfonsäuren, vorliegen. Bevorzugt sind sie in Form ihrer Salze, insbesondere der Alkali- und Erdalkalimetallsalze, wie der Natrium-, Kalium- und Calciumsalze. Sie finden bevorzugt in Form der Alkalimetallsalze Verwendung zum Färben und Bedrucken von vorzugsweise Fasermaterialien.

Die erfindungsgemäßen Disazoverbindungen der allgemeinen Formel (1) können erfindungsgemäß in der Weise hergestellt werden, daß man eine bisher noch nicht bekannte und nachfolgend beschriebene erfindungsgemäße, zweifach ankuppelbare Verbindung der allgemeinen Formel (2)

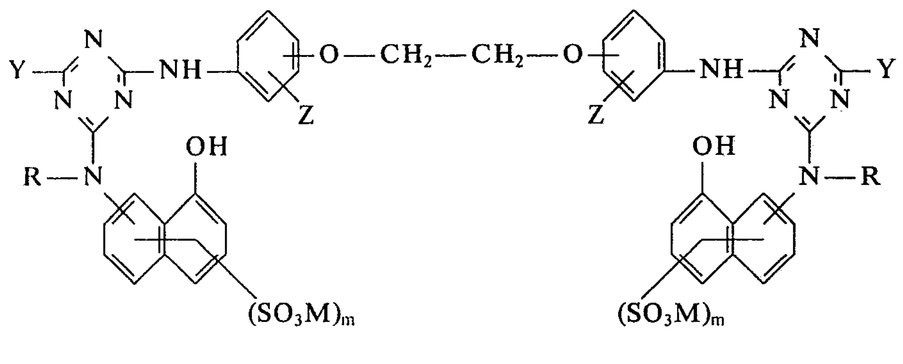

(2)

in welcher die zweifach auftretenden Formelglieder M, R, Y, Z und m jeweils eine einander identische, obengenannte Bedeutung besitzen und die Aminogruppen $-NH-$, die Gruppen Z und die Äthylendioxy-Gruppe zueinander, wie für Formel (1) angegeben, an die Benzolkerne gebunden sind, mit der äquivalenten (zweifach äquimolaren) Menge der Diazoniumverbindung eines aromatischen Amins der allgemeinen Formel (3)

$$H_2N—D—X \qquad (3)$$

in welcher D und X die obengenannten Bedeutungen haben, in an und für sich üblicher und dem Fachmann geläufiger Verfahrensweise, so in wäßrigem Medium bei einer Temperatur zwischen 0°C und 40°C, vorzugsweise zwischen 10 und 20°C, und einem pH-Wert zwischen 3,0 und 7,0, vorzugsweise zwischen 5,0 und 6,5, kuppelt, oder indem man eine an und für sich bekannte Azoverbindung der allgemeinen Formel (4)

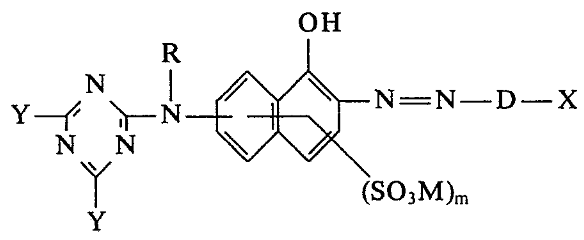
(4)

in welcher D, M, R, X, Y und m die obengenannten Bedeutungen haben, in wäßrigem Medium bei einer Temperatur zwischen 20 und 50°C, vorzugsweise zwischen 25 und 30°C, und bei einem pH-Wert zwischen 2,0 und 7,0, vorzugsweise zwischen 3,0 und 5,0, in äquivalenter Menge (d. h. 2 Mol Verbindung (4) pro Mol der nachstehenden Verbindung (5)) mit einer symmetrischen Diaminoverbindung der allgemeinen Formel (5)

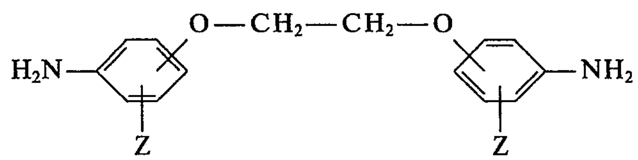
(5)

in welcher Z die obengenannte jeweils gleiche Bedeutung besitzt und die Gruppen Z an die Benzolkerne jeweils in ortho- oder jeweils in para-Stellung zum Äthylendioxy-Substituenten und die Aminogruppen und die Gruppen Z in den Benzolkernen jeweils zueinander meta-ständig gebunden sind, umsetzt.

Die obengenannten Azoverbindungen der allgemeinen Formel (4) lassen sich in an und für sich üblicher Weise durch Kupplung einer Verbindung der allgemeinen Formel (6)

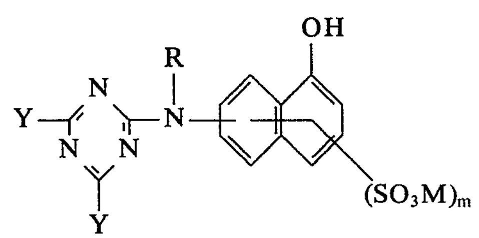
(6)

in welcher M, R, Y und m die obengenannten Bedeutungen haben, mit der Diazoniumverbindung eines Amins der obengenannten allgemeinen Formel (3) herstellen.

Die vorliegende Erfindung betrifft weiterhin, wie bereits erwähnt, neue Verbindungen der obengenannten allgemeinen Formel (2) sowie deren Herstellung und Verwendung als Kupplungskomponenten, vorzugsweise bivalente Kupplungskomponenten, zur Synthese von Azoverbindungen, vorzugsweise Disazoverbindungen. Sie können in erfindungsgemäßer Weise hergestellt werden, indem man eine Verbindung der oben genannten allgemeinen Formel (6) mit der äquivalenten Mengen eines Diamins der obigen allgemeinen Formel (5) bei einer Temperatur zwischen 5 und 50°C, vorzugsweise zwischen 10 und 25°C, und einem pH-Wert zwischen 3,5 und 7,0, bevorzugt zwischen 4,0 und 5,5, umsetzt, oder indem man eine Diaminoverbindung der obigen allgemeinen Formel (5) mit der zweifach molaren Menge an 2,4,6-Trifluor-s-triazin oder 2,4,6-Trichlor-s-triazin in wäßrigem oder wäßrig-organischem Medium bei eine Temperatur zwischen −10°C und +15°C und einem pH-Wert zwischen 2,5 und 5,5, vorzugsweise zwischen 3,0 und 4,5, zur symmetrischen Verbindung der allgemeinen Formel (7)

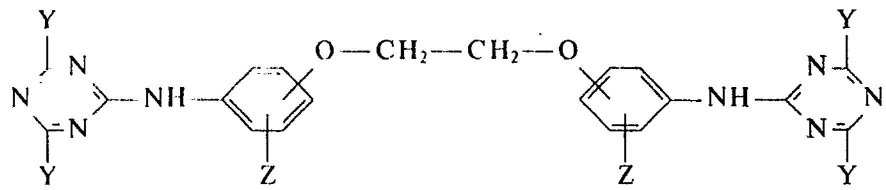

(7)

in welcher jedes Y und jedes Z die gleiche, obengenannte Bedeutung besitzt und die Äthylendioxygruppe, die Gruppen —NH— und die Gruppen Z zueinander, wie für Formel (1) angegeben, an die Benzolkerne gebunden sind, umsetzt und diese Verbindung der allgemeinen Formel (7) in an und für sich üblicher und dem Fachmann geläufiger Verfahrensweise mit einer Aminonaphtholsulfonsäure der allgemeinen Formel (8)

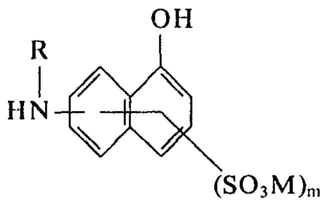

(8)

in welcher M, R und m die obengenannten Bedeutungen haben, in äquivalenter Menge (2 Mol Verbindung (8) pro Mol Verbindung (7)) kondensiert.

Die bisher nicht bekannten Diaminoverbindungen der allgemeinen Formel (5) sind in der europäischen Patentanmeldung 82 106 565.3 (Veröffentlichungs-Nr. 0 071 168 A) vom 21. Juli 1982 beschrieben. Sie lassen sich herstellen, indem man von einem Äthylenglykol-bis-anilin-äther ausgeht und diesen analog bekannten Verfahrensweisen zur N,N'-Bis-acetylamino-Verbindung acetyliert, in diese wiederum mittels Chlorsulfonsäure und Thionylchlorid, wie zum Beispiel in Houben-Weyl, Methoden der Organischen Chemie, Band IX, S. 578 (1955), beschrieben, vorzugsweise bei einer Temperatur zwischen 40 und 100°C, in die beiden Benzolkerne jeweils eine Sulfochloridgruppe einführt, diese Sulfochloridgruppen mit Alkalisulfit, insbesondere Natriumsulfit, in Gegenwart von Alkalihydroxid in wäßriger Lösung bei einer Temperatur zwischen 0 und 60°C und einem pH-Wert zwischen 7 und 10 zu den Sulfinsäuren reduziert, diese wiederum mit Äthylenoxid in Gegenwart von wäßriger Schwefelsäure oder mit Äthylenchlorhydrin, beispielsweise analog dem in der belgischen Patentschrift 747 418 beschriebenen Verfahren, zu den β-Hydroxyäthylsulfonyl-Verbindungen oxäthyliert und diese anschließend mittels einer anorganischen Säure entacetyliert. Diese so erhaltenen Diaminoverbindungen mit den β-Hydroxyäthylsulfonyl-Gruppen entsprechen somit den Verbindungen der allgemeinen Formel (5), in welcher jedoch hier der Formelrest Z die β-Hydroxyäthylsulfonyl-Gruppe ist. Diese β-Hydroxyäthylsulfonyl-Gruppen können analog bekannten Verfahrensweisen in die Gruppen Z mit den für Formel (1) genannten Bedeutungen übergeführt werden, beispielsweise durch Veresterung mit einem Sulfatierungsmittel in die entsprechenden β-Sulfatoäthylsulfonyl-Gruppen.

Die erfindungsgemäß erhaltenen Verbindungen der allgemeinen Formel (2) können entweder direkt in Lösung, d. h. mit dem Syntheseansatz nach erfolgter Synthese, oder nach Zwischenisolierung zur Herstellung von Azoverbindungen, insbesondere zur Herstellung der erfindungsgemäßen Disazoverbindungen der allgemeinen Formel (1), als Kupplungskomponenten verwendet werden. Die Isolierung der Verbindungen der allgemeinen Formel (2) aus ihren Syntheselösungen erfolgt in an und für sich üblicher und dem Fachmann geläufiger Weise durch Aussalzen mittels Elektrolyten, wie beispielsweise Natrium- oder Kaliumchlorid, oder durch Sprühtrocknung.

Die erfindungsgemäß hergestellten Disazoverbindungen der allgemeinen Formel (1) werden ebenfalls nach allgemein bekannten Methoden abgeschieden und isoliert, so beispielsweise durch Aussalzen aus dem Reaktionsmedium mittels Elektrolyten, wie beispielsweise Natriumchlorid oder Kaliumchlorid, oder durch Eindampfen, wie Sprühtrocknen. In manchen Fällen kann es auch wünschenswert sein, die Lösung der gebildeten erfindungsgemäßen Disazoverbindungen, gegebenenfalls nach Zusatz von Puffersubstanzen und/oder nach eventuellem Konzentrieren, direkt als Flüssigpräparation der färberischen Verwendung zuzuführen.

Die erfindungsgemäßen Disazoverbindungen besitzen wertvolle Farbstoffeigenschaften, die infolge ihrer Reste Z und der gegebenenfalls vorhandenen Reste X faserreaktive Eigenschaften aufweisen. Die neuen Verbindungen werden bevorzugt zum Färben (im allgemeinen Sinne) von hydroxy-, amino- oder carbamidgruppenhaltigen Materialien verwendet. Die vorliegende Erfindung betrifft somit auch die Verwendung der Verbindungen der allgemeinen Formel (1) zum Färben und Bedrucken dieser Materialien bzw. Verfahren zum Färben und Bedrucken solcher Materialien in an und für sich üblichen Verfahrensweisen, bei welchen eine Verbindung der allgemeinen Formel (1) als Farbmittel eingesetzt wird. Bevorzugt kommen die Materialien in Form von Fasermaterialien zur Anwendung, insbesondere in Form von Textilfasern.

Hydroxygruppenhaltige Materialien sind natürliche oder synthetische hydroxygruppenhaltige Materialien, wie beispielsweise Cellulosefasermaterialien oder deren Regeneratprodukte oder Polyvinylalkohole. Cellulosefasermaterialien sind vorzugsweise Baumwolle, aber auch andere Pflanzenfasern, wie Leinen, Hanf, Jute und Ramiefasern oder regenerierte Cellulosefasern, wie beispielsweise Zellwolle und Viskosekunstseide, ebenso Papier.

Carbonamidgruppenhaltige Materialien sind beispielsweise synthetische und natürliche Polyamide und Polyurethane, insbesondere in Form der Fasern, beispielsweise Wolle und andere Tierhaare, Seide, Leder, Polyamid-6,6, Polyamid-6, Polyamid-11 und Polyamid-4.

Mit den erfindungsgemäßen Disazoverbindungen erhält man beispielsweise auf Cellulosefasern nach dem Ausziehverfahren unter Verwendung verschiedenster Alkalizusätze aus langer Flotte Färbungen mit sehr guter Farbausbeute. Die Verbindungen der allgemeinen Formel (1) färben Cellulosefasern unter Verwendung der bekannten Klotzverfahren ebenfalls mit ausgezeichneten Farbausbeuten, wobei diese Verbindungen mittels Alkalizusätzen durch Verweilen bei Raumtemperatur, durch Dämpfen oder mit Trockenhitze fixiert werden können.

Nach den üblichen Druckverfahren für Cellulosefasern, so einphasig, beispielsweise durch Bedrucken mit einer Natriumbicarbonat, Natriumtrichloracetat oder ein anderes säurebindendes Mittel enthaltenden Druckpaste und anschließendes Dämpfen bei 101—103°C des so bedruckten Materials, oder zweiphasig, beispielsweise durch Bedrucken mit neutraler oder schwach saurer Druckpaste und anschließende Fixierung entweder durch Hindurchführen des bedruckten Materials durch ein heißes elektrolythaltiges alkaliches Bad oder durch Überklotzen mit einer alkalischen elektrolythaltigen Klotzflotte und Verweilen, Dämpfen oder Behandlung mit Trockenhitze des überklotzten Materials, erhält man ebenfalls farbstarke Drucke mit gutem Stand der Konturen und einem klaren Weißfond. Der Ausfall der Drucke ist von wechselnden Fixierbedingungen nur wenig abhängig; die Drucke zeigen daher eine sehr befriedigende Nuancenkonstanz.

Aus anwendungstechnischen Gründen besonders bevorzugte Verbindungen der allgemeinen Formel (1) sind solche, die als faserreaktiven Rest Z die $\beta$-Sulfatoäthylsulfonylgruppe tragen, desgleichen solche Farbstoffe, welche die in der Beschreibung näher bezeichneten heterocyclischen Reaktivreste X, die über eine Gruppe —NR″— an D gebunden sind, besitzen.

Die mit den Verbindungen der allgemeinen Formel (1) erhältlichen Färbungen oder Drucke auf Cellulosefasermaterialien besitzen sehr gute Echtheiten; hiervon sind insbesondere die wichtigsten Fabrikations- und Gebrauchsechtheiten hervorzuheben, wie die Lichtechtheit, die Waschechtheit, beispielsweise bei 60°C oder 95°C, die saure und alkalische Walkechtheit, die Wasserechtheit, die Seewasserechtheit, die saure Überfärbeechtheit, die alkalische und saure Schweißechtheit sowie die Plissier-, Bügel- und Reibechtheiten.

Die Färbungen auf Polyamidfasern werden üblicherweise aus saurem Milieu ausgeführt. So kann man beispielsweise dem Färbebad Essigsäure, Essigsäure und Ammoniumacetat oder Natriumacetat zufügen, um den gewünschten pH-Wert zu erhalten. Zwecks Erreichung einer brauchbaren Egalität der Färbungen empfiehlt sich ein Zusatz an üblichen Egalisierhilfsmitteln, beispielsweise auf Basis eines Umsetzungsproduktes von Cyanurchlorid mit der dreifach molaren Menge einer Aminobenzol- und/oder einer Aminonaphthalinsulfonsäure oder auf Basis eines Umsetzungsproduktes von einem Fettamin, wie beispielsweise Stearylamin, mit einem Alkylenoxid, wie beispielsweise Äthylenoxid. Die Färbungen können üblicherweise bei Temperaturen von 60 bis 105°C, vorzugsweise im Ausziehverfahren insbesondere bei Siedetemperatur des Färbebades, oder auch in einem Druckfärbeapparat bei Temperaturen bis zu 120°C ausgeführt werden.

Die nachstehenden Beispiele dienen zur Erläuterung der Erfindung. Die darin genannten Teile sind Gewichtsteile, die Prozentangaben stellen Gewichtsprozente dar, sofern nichts anderes vermerkt. Gewichtsteile beziehen sich zu Volumenteilen wie Kilogramm zu Liter.

Die in diesen Beispielen formelmäßig beschriebenen Verbindungen sind in der Form der freien Säuren angegeben; im allgemeinen werden sie in Form ihrer Natrium- oder Kaliumsalze hergestellt und isoliert und in Form ihrer Salze zum Färben verwendet. Ebenso können die in den nachfolgenden Beispielen, insbesondere Tabellenbeispielen, in Form der freien Säure genannten Ausgangsverbindungen und Komponenten als solche oder in Form ihrer Salze, vorzugsweise Alkalimetallsalze, wie Natrium- oder Kaliumsalze, in die Synthese eingesetzt werden.

## Beispiel 1

Zu einer wäßrigen Lösung von 46,7 Teilen 1-(4,6-Dichlor-s-triazin-2-yl-amino)-8-naphthol-3,6-disulfonsäure wird bei einer Temperatur von 10 bis 15°C und einem pH-Wert von 2,5 eine neutrale Lösung von 31,1 Teilen Äthylenglykol-bis-(4-amino-2-$\beta$-sulfatoäthylsulfonyl-phenyl)-äther in 150 Teilen Wasser gegeben. Der pH des Reaktionsgemisches wird während der Umsetzung mit Natriumcarbonat bei einem Wert von 6,0 gehalten. Diese Kondensationsreaktion kann dünnschichtchromatographisch verfolgt werden. Das Reaktionsprodukt der Formel (in Form der freien Säure geschrieben)

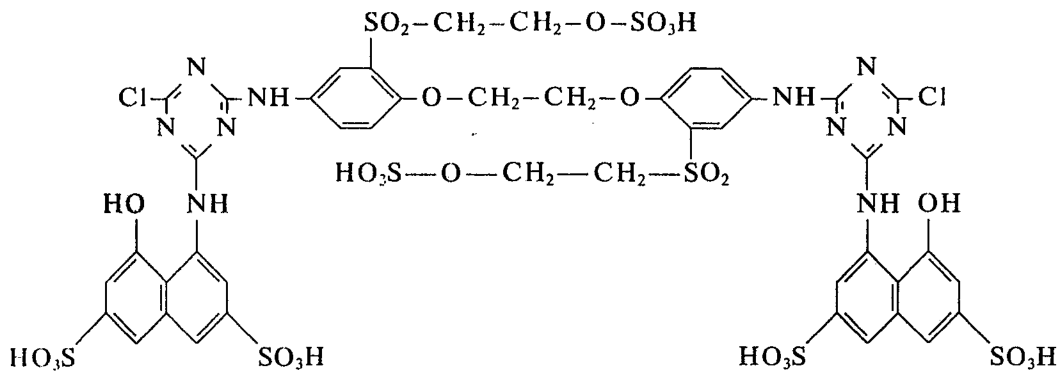

das als erfindungsgemäße Verbindung als doppelankuppelbare Kupplungskomponente zur Herstellung von Disazoverbindungen eingesetzt werden kann, kann somit durch dünnschichtchromatographischen Vergleich mit dem Ausgangsprodukt und eventuellen Nebenprodukten, ebenso durch seine Funktion als bivalente Kupplungskomponente, charakterisiert werden.

Nach Beendigung der Umsetzung wird das Kondensationsprodukt mit Natriumchlorid ausgefällt, abfiltriert, mit 10%iger wäßriger Natriumchloridlösung gewaschen und gegebenenfalls getrocknet. Es wird auf diese Weise das Natriumsalz der oben formelmäßig angegebenen Verbindung isoliert, die als Kupplungskomponente zur Herstellung von roten, insbesondere brillantroten Disazoverbindungen mit sehr guten faserreaktiven Farbstoffeigenschaften verwendet werden kann.

### Beispiel 2

Eine wäßrige Lösung von 46,7 Teilen 1-(4,6-Dichlor-s-triazin-2-yl-amino)-8-naphthol-3,6-disulfonsäure wird bei einer Temperatur von 10 bis 15°C und einem pH-Wert von 2,5 mit einer neutralen Lösung von 32,7 Teilen Äthylenglykol-bis-(4-amino-2-$\beta$-thiosulfatoäthylsulfonyl-phenyl)-äther in 200 Teilen Wasser versetzt. Der pH-Wert des Reaktionsgemisches wird auf 6,0 gestellt. Diese Kondensationsreaktion kann dünnschichtchromatographisch verfolgt werden. Das Reaktionsprodukt der Formel (in Form der freien Säure geschrieben)

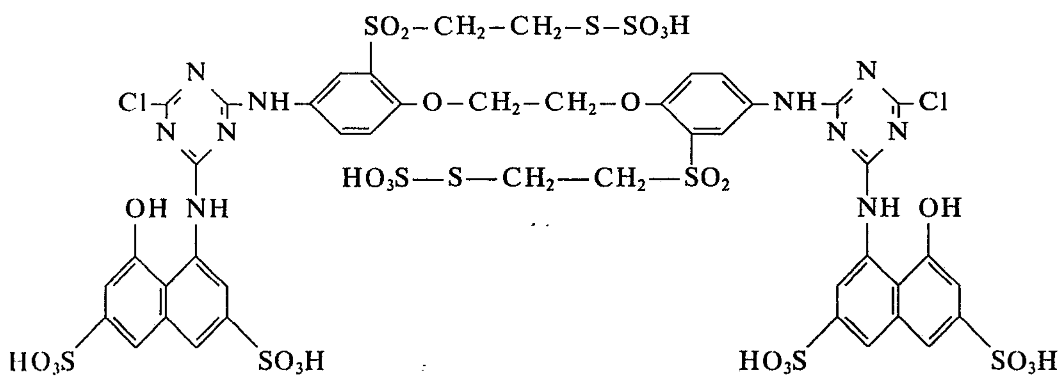

das als erfindungsgemäße Verbindung als doppelankuppelbare Kupplungskomponente zur Herstellung von Disazoverbindungen verwendet werden kann, läßt sich somit durch dünnschichtchromatographischen Vergleich mit dem Ausgangsprodukt und eventuellen Nebenprodukten, ebenso durch seine Funktion als bivalente Kupplungskomponente, charakterisieren.

Nach Beendigung der Kondensationsreaktion wird die erfindunsgemäße Verbindung mittels Natriumchlorid ausgesalzen, abfiltriert und mit 20%iger wäßriger Natriumchloridlösung gewaschen und gegebenenfalls getrocknet. Es wird auf diese Weise das Natriumsalz oben formelmäßig angegebener Verbindung isoliert, die als Kupplungskomponente zur Herstellung von roten, insbesondere brillantroten, Disazoverbindungen mit sehr guten Farbstoffeigenschaften verwendet werden kann.

### Beispiel 3

Eine wäßrige Lösung mit einem pH-Wert von 3,5 von 38,7 Teilen 2-(4,6-Dichlor-s-triazin-2-yl-amino)-5-naphthol-7-sulfonsäure werden bei einer Temperatur von 10—15°C mit einer neutralen Lösung von 31,1 Teilen Äthylenglykol-bis-(4-amino-2-$\beta$-sulfatoäthylsulfonyl-phenyl)-äther in 150 Teilen Wasser versetzt. Das Reaktionsgemisch wird mit Natriumcarbonat auf einen pH-Wert von 6,0 gestellt

und während der Umsetzung bei diesem pH-Wert gehalten. Die Kondensationsreaktion kann dünnschichtchromatographisch verfolgt werden. Das Reaktionsprodukt der Formel (in Form der freien Säure geschrieben)

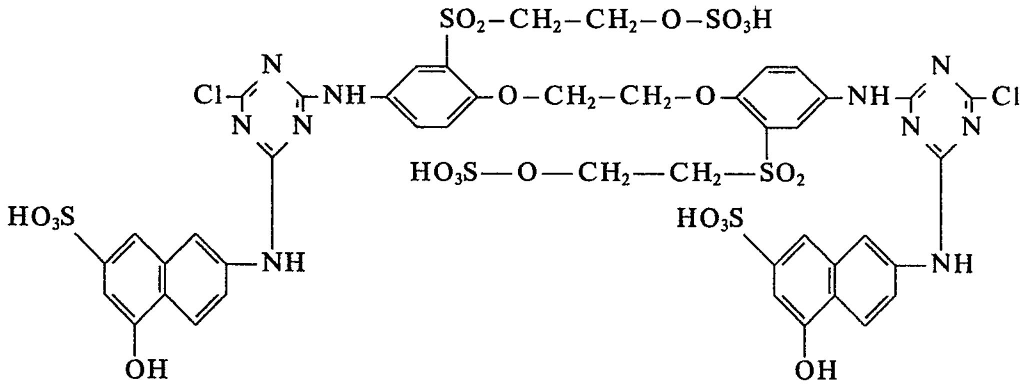

das als erfindungsgemäße Verbindung als doppelankuppelbare Kupplungskomponente zur Herstellung von Disazoverbindungen verwendet werden kann, läßt sich somit durch dünnschichtchromatographischen Vergleich mit dem Ausgangsprodukt und eventuellen Nebenprodukten, ebenso durch seine Funktion als bivalente Kupplungskomponente, charakterisieren.

Nach erfolgter Umsetzung wird die erfindungsgemäße Verbindung mit Natriumchlorid ausgesalzen, abfiltriert und mit wäßriger Natriumchloridlösung gewaschen und gegebenenfalls getrocknet. Es wird auf diese Weise das Natriumsalz oben formelmäßig angegebener Verbindung isoliert, die als Kupplungskomponente zur Herstellung von orangefarbigen Disazoverbindungen mit sehr guten faserreaktiven Farbstoffeigenschaften verwendet werden kann.

Beispiel 4

Eine Lösung mit einem pH-Wert von 6,8 von 31,1 Teilen Äthylenglykol-bis-(4-amino-2-$\beta$-sulfatoäthylsulfonyl-phenyl)-äther in 100 Teilen Wasser mit einer Temperatur von 5°C wird in eine Suspension von 18,8 Teilen Cyanurchlorid in 30 Teilen Wasser und 30 Teilen Eis eingerührt. Bis zur Beendigung der Reaktion wird die Temperatur des Gemisches bei 5°C und der pH-Wert bei 3,0 bis 3,5 gehalten. Die klare Lösung dieses Kondensationsproduktes wird sodann mit einer Lösung mit einem pH-Wert von 6,8 von 25,3 Teilen 2-N-Methylamino-5-naphthol-7-sulfonsäure versetzt, das Reaktionsgemisch auf einen pH-Wert von 6,0 gestellt, auf 60°C erhitzt und bei diesem pH-Wert und dieser Temperatur 2 Stunden lang gehalten. Die Kondensationsreaktion kann dünnschichtchromatographisch verfolgt werden. Das Reaktionsprodukt der Formel (in Form der freien Säure geschrieben)

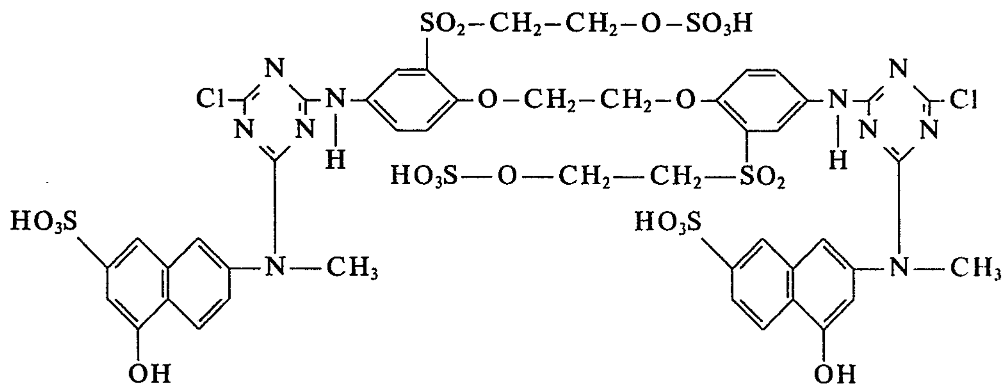

das als erfindungsgemäße Verbindung als doppelankuppelbare Kupplungskomponente zur Herstellung von Disazoverbindungen verwendet werden kann, läßt sich somit durch dünnschichtchromatographischen Vergleich mit dem Ausgangsprodukt und eventuellen Nebenprodukten, ebenso durch seine Funktion als bivalente Kupplungskomponente, charakterisieren.

Nach erfolgter Umsetzung kann die erfindungsgemäße Verbindung in üblicher Weise, wie beispielsweise in einem der vorherigen Beispiele beschrieben, isoliert und zu Endprodukten (Disazoverbindungen) weiterverarbeitet werden.

Die Weiterverarbeitung ist natürlich auch in Form der erhaltenen Syntheselösung, d. h. ohne Zwischenisolierung, möglich. Mit ihr als Kupplungskomponente kann man durch Umsetzung mit anderen üblichen Diazokomponenten orangefarbene bis rote Disazoverbindungen erhalten, die sehr gute faserreaktive Farbstoffeigenschaften besitzen.

Beispiel 5

Zu einer Lösung mit einem pH-Wert von 6,9 von 31,1 Teilen Äthylenglykol-bis-(4-amino-2-β-sulfatoäthylsulfonyl-phenyl)-äther in 75 Teilen Wasser werden bei einer Temperatur von 5°C 13,6 Teile Cyanurfluorid unter gutem Rühren zugetropft, wobei der pH-Wert mittels Natriumhydrogencarbonat zwischen 3,0 und 3,5 gehalten wird. Die klare Lösung wird nach 10minütigem Rühren mit einer Suspension mit einem pH-Wert von 6,7 von 23,9 Teilen 2-Amino-5-naphthol-7-sulfonsäure in 150 Teilen Wasser versetzt. Das Reaktionsgemisch wird sodann auf einen pH-Wert von 5,5 gestellt, bei diesem pH zunächst 2 Stunden bei etwa 20°C, anschließend eine Stunde bei 50°C gerührt. Die Kondensationsreaktion kann dünnschichtchromatographisch verfolgt werden. Das Reaktionsprodukt der Formel (in Form der freien Säure geschrieben)

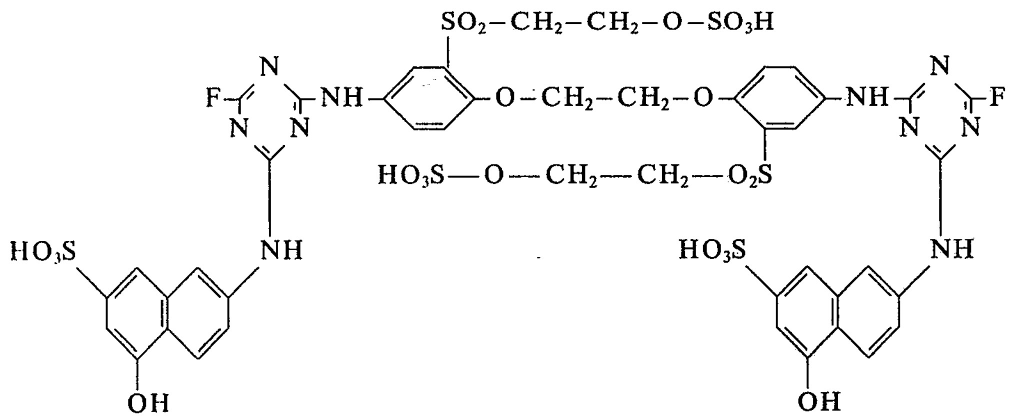

das als erfindungsgemäße Verbindung als doppelankuppelbare Kupplungskomponente zur Herstellung von Disazoverbindungen verwendet werden kann, läßt sich somit durch dünnschichtchromatographischen Vergleich mit dem Ausgangsprodukt und eventuellen Nebenprodukten, ebenso durch seine Funktion als bivalente Kupplungskomponente, charakterisieren.

Die erfindungsgemäße Verbindung wird in üblicher Weise, beispielsweise gemäß den Angaben der obigen Beispiele, isoliert; sie kann auch ohne Zwischenisolierung in Form der angefallenen Syntheselösung weiterverarbeitet werden, so zur Herstellung von orangefarbigen Disazoverbindungen durch Kupplung mit der zweifach molaren Menge einer Diazokomponente. Die so erfindungsgemäß erhältlichen Disazoverbindungen besitzen sehr gute faserreaktive Farbstoffeigenschaften.

Beispiel 6

Zu einer Lösung mit einem pH-Wert von 6,9 von 31,1 Teilen Äthylenglykol-bis-(2-amino-4-β-sulfatoäthylsulfonyl-phenyl)-äther in 75 Teilen Wasser werden bei 5°C 13,6 Teile Cyanurfluorid unter Rühren zugetropft, wobei der pH-Wert des Reaktionsgemisches mittels Natriumhydrogencarbonat zwischen 3,0 und 3,5 gehalten wird. Nach 15minütigem Nachrühren wird die Lösung mit einer Suspension mit einem pH-Wert von 6,7 von 31,9 Teilen 1-Amino-8-naphthol-3,6-disulfonsäure in 60 Teilen Wasser versetzt. Dieses Reaktionsgemisch wird bei einem pH-Wert von 5,5 zunächst 2 Stunden bei einer Temperatur von 20°C, anschließend eine Stunde bei 50°C gerührt. Die Kondensationsreaktion kann dünnschichtchromatographisch verfolgt werden. Das Reaktionsproduk der Formel (in Form der freien Säure geschrieben)

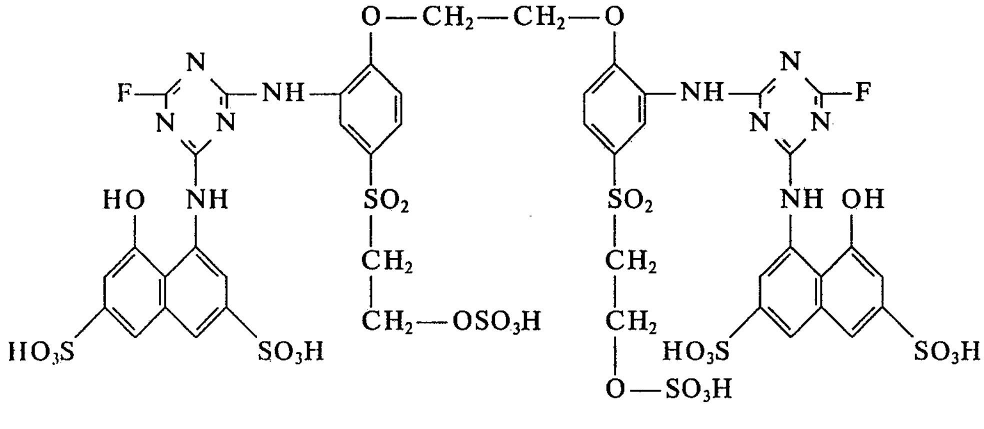

das als erfindungsgemäße Verbindung als doppelankuppelbare Kupplungskomponente zur Herstellung von Disazoverbindungen verwendet werden kann, läßt sich somit durch dünnschichtchromatographischen Vergleich mit dem Ausgangsprodukt und eventuellen Nebenprodukten, ebenso durch seine Funktion als bivalente Kupplungskomponente, charakterisieren.

Es wird in üblicher Weise isoliert und liefert nach der Umsetzung mit üblichen Diazokomponenten rote Disazoverbindungen mit sehr guten faserreaktiven Farbstoffeigenschaften.

## Beispiel 7

17,3 Teile Anilin-2-sulfonsäure werden in wäßriger Lösung in üblicher Weise diazotiert und mit 74,1 Teilen der erfindunsgemäßen Verbindung (Kupplungskomponente) des Beispiels 1, beispielsweise in Form einer wäßrigen Suspension, versetzt. Der pH dieses Kupplungsgemisches wird sodann mit Natriumcarbonat auf einen Wert zwischen 6,0 und 6,5 gestellt und während der gesamten Kupplungsreaktion in diesem Bereich gehalten. Nach Beendigung der Reaktion wird die erfindungsgemäße Disazoverbindung durch Aussalzen mittels Natriumchlorid oder durch Sprühtrocknung isoliert.

Es wird in dunkelrotes, elektrolythaltiges Pulver des Alkalimetallsalzes, vorwiegend Natriumsalzes, der Verbindung der Formel

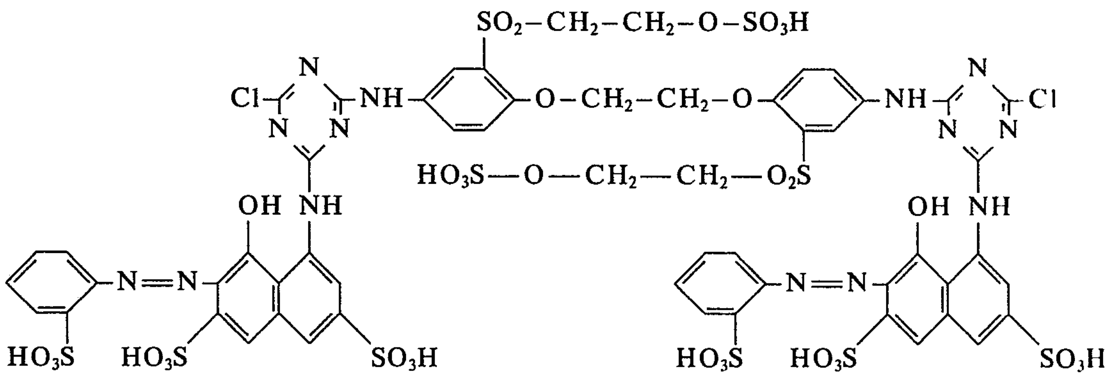

erhalten. Diese erfindungsgemäße Disazoverbindung besitzt sehr gute Farbstoffeigenschaften und färbt nach den für Reaktivfarbstoffe üblichen Färbe- und Druckverfahren Cellulosefasermaterialien in Gegenwart von Alkalien, wie Natriumbicarbonat, Natriumcarbonat oder wäßrigem Natriumhydroxyd, in vollen klaren Rottönen mit sehr guten Gebrauchs- und Fabrikationsechtheiten, insbesondere sehr guten Naßechtheiten und sehr guten Lichtechtheiten.

## Beispiel 8

30,3 Teile 2-Naphthylamin-1,5-disulfonsäure werden in wäßriger Lösung in üblicher Weise diazotiert. Zur Suspension der Diazoniumverbindung gibt man sodann 80,3 Teile der im Beispiel 2 beschriebenen erfindungsgemäßen Verbindung (Kupplungskomponente), stellt den pH dieses Kupplungsgemisches mit Natriumcarbonat auf einen Wert zwischen 6,0 und 6,5 ein und führt die Kupplungsreaktion durch mehrstündiges Rühren innerhalb dieses pH-Bereiches zu Ende.

Die erfindungsgemäße Disazoverbindung wird mit Natriumchlorid ausgesalzen, abgesaugt, getrocknet und vermahlen. Es wird ein rotes, elektrolythaltiges Pulver des Alkalimetallsalzes, vorwiegend Natriumsalzes, der Verbindung der Formel

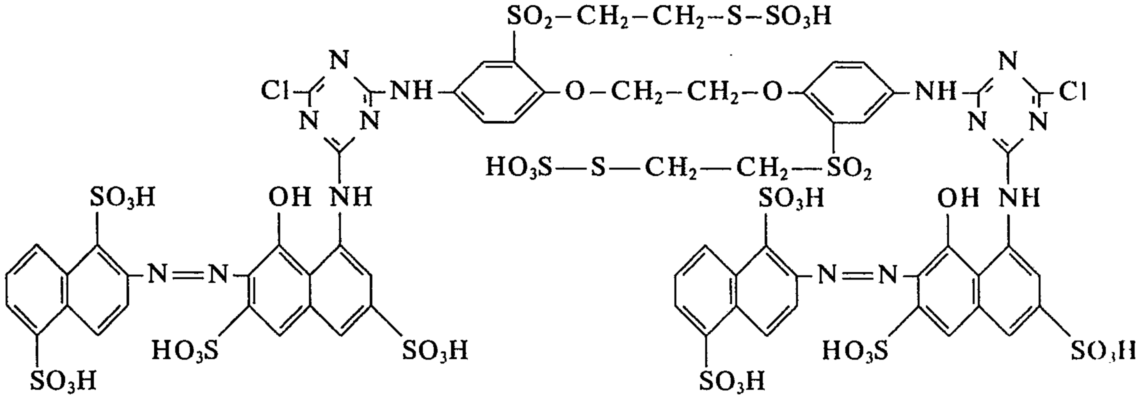

erhalten. Diese erfindungsgemäße Disazoverbindung zeigt sehr gute Farbstoffeigenschaften und färbt nach den für Reaktivfarbstoffe üblichen Färbe- und Druckmethoden und Fixierverfahren Cellulosefasermaterialien, wie Baumwolle, in brillanten Rottönen; diese Färbungen und Drucke besitzen sehr gute Gebrauchs- und Fabrikationsechtheiten, wie insbesondere sehr gute Naßechtheiten und Lichtechtheiten.

Beispiel 9

38,3 Teile 2-Naphthylamin-1,5,7-trisulfonsäure werden in üblicher Weise diazotiert. Die wäßrige Lösung dieser Diazoniumverbindung wird unter Rühren mit 69,8 Teilen der erfindungsgemäßen Verbindung (Kupplungskomponente) des Beispieles 3 versetzt und der pH-Wert dieses Kupplungsgemisches mittels Natriumcarbonat auf 5,5 eingestellt. Nach mehrstündigem Rühren bei diesem pH-Wert ist die Kupplungsreaktion zu Ende geführt. Die erfindungsgemäße Disazoverbindung wird mit Natriumchlorid ausgesalzen, abfiltriert, mit wäßriger Natriumchloridlösung nachgewaschen, getrocknet und vermahlen. Man erhält ein rotes elektrolythaltiges Pulver des Alkalimetallsalzes, vorwiegend Natriumsalzes, der Verbindung der Formel

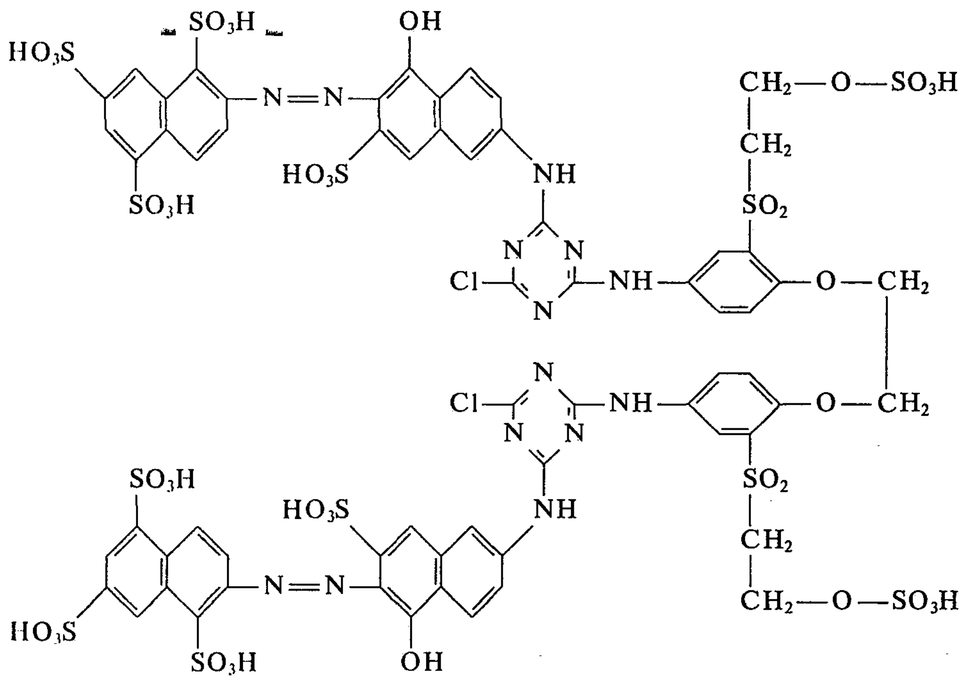

Diese Disazoverbindung besitzt sehr gute Farbstoffeigenschaften und färbt nach den für Reaktivfarbstoffe üblichen Färbe- und Druckmethoden in Gegenwart von Alkalien, wie Natriumcarbonat, Natriumbicarbonat oder wäßrigem Natriumhydroxid, Cellulosematerialien in klaren Orangetönen, die insbesondere gute Lichtechtheiten und sehr gute Naßechtheiten aufweisen.

Beispiel 10

20,3 Teile 4-Amino-anisol-3-sulfonsäure werden in üblicher Weise in Wasser diazotiert und sodann mit 71,2 Teilen der im Beispiel 4 beschriebenen erfindungsgemäßen Verbindung (Kupplungskomponente) unter Rühren versetzt. Der pH dieses Kupplungsgemisches wird mittels Natriumcarbonat auf einen Wert zwischen 6,0 und 6,5 eingestellt und bis zur Beendigung der Kupplungsreaktion gehalten.

Die erfindungsgemäße Disazoverbindung wird durch Sprühtrocknung oder durch Aussalzen mit Natriumchlorid isoliert. Es wird ein rotes elektrolythaltiges Pulver des Alkalimetallsalzes, vorwiegend Natriumsalzes, der Verbindung der Formel

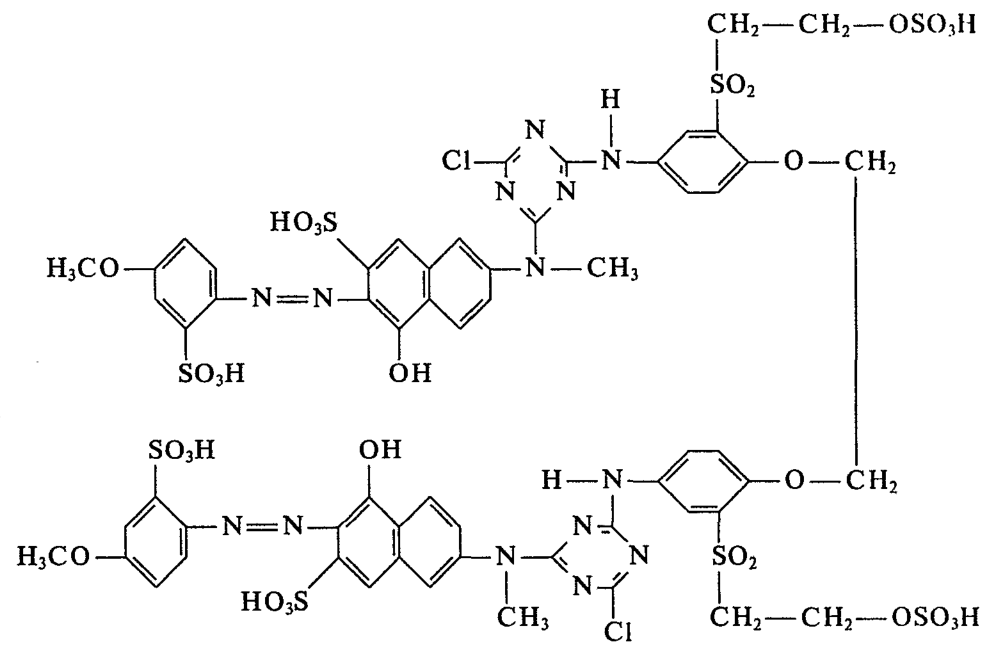

erhalten. Die erfindungsgemäße Verbindung zeigt sehr gute faserreaktive Farbstoffeigenschaften und färbt die in der Beschreibung genannten Materialien, vorzugsweise Cellulosefasermaterialien, nach den für faserreaktive Farbstoffe üblichen Färbe- und Druckmethoden und Fixierverfahren, so in Gegenwart von Alkalien, wie beispielsweise Natriumcarbonat oder wäßrigem Natriumhydroxid, in scharlachroten Tönen. Die mit der erfindunsgemäßen Disazoverbindung erhältlichen Färbungen und Drucke zeigen sehr gute Gebrauchs- und Fabrikationsechtheiten, wie insbesondere gute Lichtechtheiten und sehr gute Naßechtheiten.

## Beispiel 11

Man verfährt zur Herstellung einer erfindungsgemäßen Disazoverbindung gemäß der Verfahrensweise des Beipieles 9, setzt jedoch anstelle der dort verwendeten Kupplungskomponente des Beispiels 3 die äquivalente Menge der im Beispiel 5 beschriebenen erfindungsgemäßen Verbindung (Kupplungskomponente) in die Kupplungsreaktion ein. Nach Beendigung der Kupplungsreaktion und üblicher Aufarbeitung erhält man das Alkalimetallsalz, wie Natriumsalz, der Verbindung der Formel

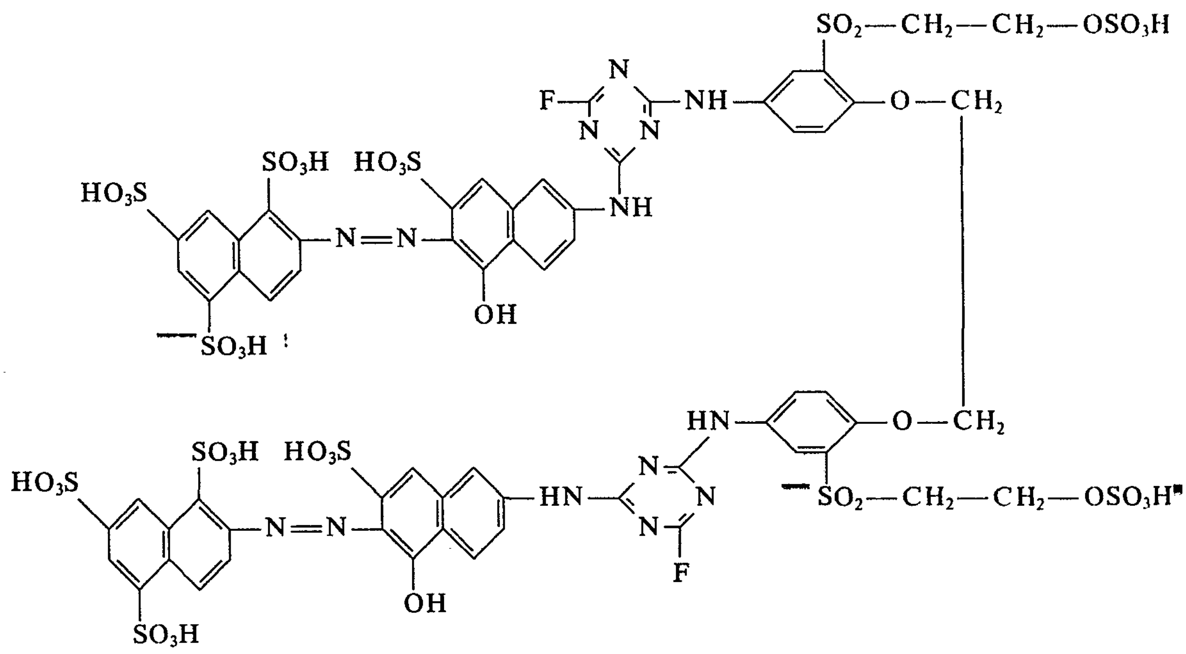

Diese erfindungsgemäße Disazoverbindung zeigt sehr gute faserreaktive Farbstoffeigenschaften und liefert auf den in der Beschreibung genannten Materialien, wie insbesondere Cellulosefasermaterialien, beispielsweise Baumwolle, nach den für faserreaktive Farbstoffe in der Technik üblichen Applikations- und Fixiermethoden Färbungen und Drucke in klaren Orangetönen mit sehr guten Naßechtheiten und guten Lichtechtheiten.

Beispiel 12

9,3 Teile Anilin werden in üblicher Weise in Wasser diazotiert. Die wäßrige Lösung der Diazoniumverbindung wird sodann mit 77 Teilen der im Beispiel 6 beschriebenen erfindungsgemäßen Verbindung (Kupplungskomponente) versetzt, und die Kupplungsreaktion wird bei einem pH-Wert von 6,0 durchgeführt. Die Disazoverbindung wird sodann in üblicher Weise isoliert, beispielsweise durch Aussalzen mit Natriumchlorid, Abfiltration und Waschen des Produktes mit wäßriger Natriumchloridlösung. Nach Trocknen und Vermahlen erhält man ein rotes elektrolythaltiges Pulver des Natriumsalzes der Verbindung der Formel

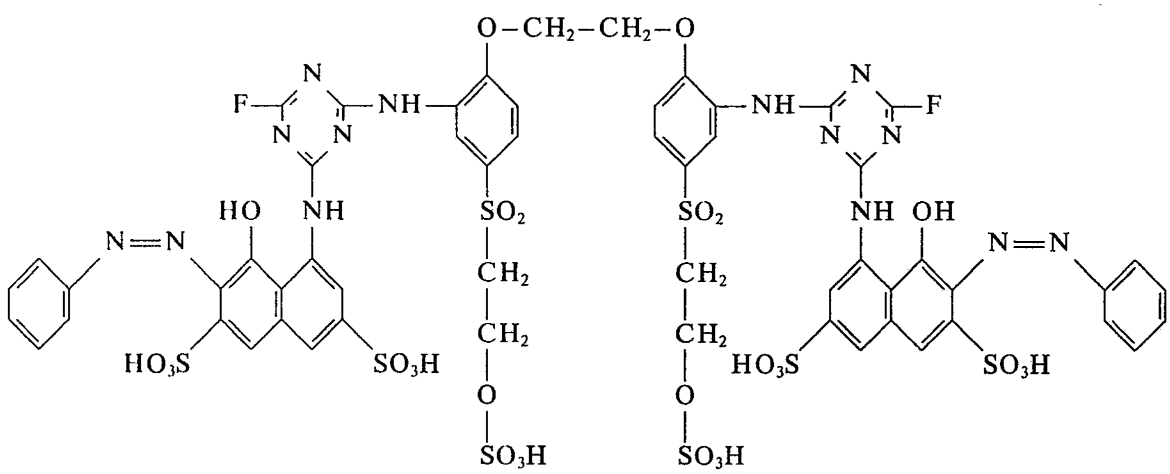

Diese zeigt sehr gute Farbstoffeigenschaften und färbt nach den für Reaktivfarbstoffe üblichen Färbe- und Druckmethoden und Fixierverfahren Cellulosefasermaterialien, wie Baumwolle, in klaren Rottönen mit sehr guten Gebrauchs- und Fabrikationsechtheiten, wie insbesondere sehr gute Naßechtheiten und einer guten Lichtechtheit.

Beispiel 13

18,7 Teile 1-Amino-4-methyl-benzol-2-sulfonsäure werden in 150 Teilen Wasser unter Zugabe von Natriumcarbonat bei einem pH-Wert von 6,8 gelöst und mit 20 Teilen einer 5n-Natriumnitritlösung versetzt. Die Suspension wird in eine Mischung aus 30 Teilen 31%iger Salzsäure und 100 Teilen Eis eingerührt. Man rührt noch eine Stunde und zerstört überschüssige salpetrige Säure mit Amidosulfonsäure. Anschließend gibt man eine auf 5°C abgekühlte, 46,7 Teile 1-(4,6-Dichlor-s-triazin-2-ylamino)-8-naphthol-4,6-disulfonsäure enthaltende wäßrige Suspension vom pH-Wert 3,5 hinzu; die Umsetzung dieses Reaktionsgemisches erfolgt bei einer Temperatur von 10°C und einem pH-Wert von 5,5. Man fügt sodann eine auf den pH-Wert von 6,8 eingestellte Lösung von 31,1 Teilen Äthylenglykol-bis-(4-amino-2-$\beta$-sulfatoäthylsulfonyl-phenyl)-äther in 150 Teilen Wasser hinzu. Der pH-Wert des Reaktionsgemisches wird mit Natriumcarbonat auf einen Wert von 5,5 gestellt. Sobald die Reaktionstemperatur bei 20°C konstant bleibt, erwärmt man das Gemisch bei einem pH-Wert von 5,5 noch eine Stunde auf 50°C.

Man kühlt auf Raumtemperatur ab und fällt das Reaktionsprodukt durch Zugabe von Natriumchlorid aus. Nach Trocknen und Vermahlen erhält man ein dunkelrotes elektrolythaltiges Pulver, welches das Alkalimetallsalz, vorwiegend Natriumsalz, der Verbindung der Formel

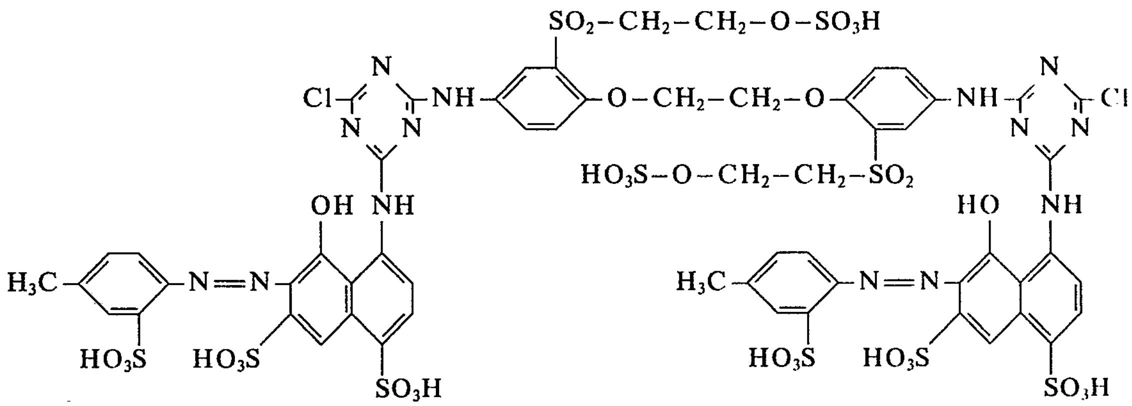

enthält. In Gegenwart von Alkalien werden mit dieser Disazoverbindung nach den üblichen Färbe- und Druckverfahren auf Cellulosefasermaterialien klare Rotfärbungen mit ausgezeichneten Gebrauchs- und Fabrikationsechtheiten, insbesondere sehr guten Naßechtheiten und sehr guten Lichtechtheiten erzielt.

### Beispiele 14 bis 34

In den nachfolgenden Tabellenbeispielen sind weitere erfindungsgemäße Disazoverbindungen entsprechend der allgemeinen Formel (1) mit Hilfe ihrer Ausgangsverbindungen beschrieben. Sie lassen sich in erfindungsgemäßer Weise, beispielsweise gemäß den Verfahrensweisen der obigen Ausführungsbeispiele 7 bis 13, aus diesen Ausgangsverbindungen herstellen.

Diese erfindungsgemäßen Disazoverbindungen besitzen ebenfalls sehr gute faserreaktive Farbstoffeigenschaften und färben nach den in der Technik üblichen Applikations- und Fixiermethoden, insbesondere solchen für faserreaktive Farbstoffe, die in der Beschreibung genannten Materialien, wie insbesondere natürliche und regenerierte Cellulosefasermaterialien, vorzugsweise Baumwolle, in farbstarken und echten (in den Tabellenbeispielen angegebenen) Tönen.

| Beispiel | Diazokomponente | Azokomponente | 2,4,6-Halogen-s-triazin | Äthylenglykol-bis-aryläther-derivat | Farbton auf Cellulose |
|---|---|---|---|---|---|
| 14 | Anilin | 1-Amino-8-naphthol-3,6-disulfonsäure | Cyanurchlorid | Äthylenglykol-bis-(4-amino-2-β-sulfatoäthylsulfonyl-phenyl)-äther | rot |
| 15 | m-Toluidin | 1-Amino-8-naphthol-4,6-disulfonsäure | Cyanurfluorid | desgl. | rot |
| 16 | 4-Amino-anisol-3-sulfonsäure | desgl. | Cyanurchlorid | desgl. | rotviolett |
| 17 | 4-Chlor-anilin-2-sulfonsäure | 1-Amino-8-naphthol-3,6-disulfonsäure | desgl. | desgl. | rot |
| 18 | Anilin-3-sulfonsäure | 2-Amino-8-naphthol-6-sulfonsäure | desgl. | desgl. | orange |
| 19 | 3-Chlor-4-methylanilin-6-sulfonsäure | 1-Amino-8-naphthol-3,6-disulfonsäure | desgl. | desgl. | rot |

Fortsetzung

| Beispiel | Diazokomponente | Azokomponente | 2,4,6-Halogen-s-triazin | Äthylenglykol-bis-aryläther-derivat | Farbton auf Cellulose |
|---|---|---|---|---|---|
| 20 | 3-Methyl-4-chlor-anilin-6-sulfonsäure | 1-Amino-8-naphthol-3,6-disulfonsäure | Cyanurchlorid | Äthylenglykol-bis-(2-amino-4-β-sulfato-äthyl-sulfonyl-phenyl)-äther | rot |
| 21 | Anilin-4-sulfonsäure | desgl. | desgl. | desgl. | rot |
| 22 | 2-Naphthylamin-1,5-disulfonsäure | 2-Amino-5-naphthol-7-sulfonsäure | Cyanurfluorid | Äthylenglykol-bis-(4-amino-2-β-methan-sulfonyl-oxyäthylsulfonyl-phenyl)-äther | orange |
| 23 | 1-Amino-3-(4,6-dichlor-s-triazin-2-yl-amino)-benzol-6-sulfonsäure | desgl. | Cyanurchlorid | Äthylenglykol-bis-(4-amino-2-β-sulfatoäthyl-sulfonyl-phenyl)-äther | orange |
| 24 | 1-Amino-4-(4-chlor-6-methoxy-s-triazin-2-ylamino)-benzol-6-sulfonsäure | desgl. | desgl. | desgl. | scharlach |
| 25 | 1-Amino-3-[4-chlor-6-(N-äthyl-phenyl-amino)-s-triazin-2-ylamino]-benzol-6-sulfonsäure | 1-Amino-8-naphthol-3,6-disulfonsäure | desgl. | desgl. | rot |
| 26 | 1-Amino-3-[4-chlor-6-(3-sulfo-phenyl-amino)-s-triazin-2-ylamino]-benzol-6-sulfonsäure | desgl. | desgl. | desgl. | rot |
| 27 | (4-Amino-phenyl)-(β-sulfatoäthyl)-sulfon | desgl. | desgl. | desgl. | rot |
| 28 | (3-Amino-phenyl)-(β-sulfatoäthyl)-sulfon | 1-Amino-8-naphthol-4,6-disulfonsäure | desgl. | desgl. | rot |
| 29 | 6-β-Sulfatoäthyl-sulfonyl-2-naphthyl-amin-1-sulfonsäure | desgl. | desgl. | desgl. | rot |
| 30 | desgl. | 2-Amino-5-naphthol-7-sulfonsäure | desgl. | desgl. | orange |

Fortsetzung

| Beispiel | Diazokomponente | Azokomponente | 2,4,6-Halogen-s-triazin | Äthylenglykol-bis-aryläther-derivat | Farbton auf Cellulose |
|---|---|---|---|---|---|
| 31 | 2-Amino-4-β-sulfatoäthyl-sulfonyl-anisol | 2-Methylamino-5-naphthol-7-sulfonsäure | Cyanurfluorid | Äthylenglykol-bis-(4-amino-2-β-sulfatoäthyl-sulfonyl-phenyl)-äther | scharlach |
| 32 | 1-Amino-3-[4-fluor-6-(3-sulfo-phenyl-amino)-s-triazin-2-ylamino]-benzol-6-sulfonsäure | desgl. | desgl. | desgl. | orange |
| 33 | 1-Amino-3-[4-fluor-6-(2-sulfo-phenyl-amino)-s-triazin-2-ylamino]-benzol-6-sulfonsäure | 1-Amino-8-naphthol-3,6-disulfonsäure | desgl. | desgl. | rot |
| 34 | Anilin-2,5-disulfonsäure | desgl. | desgl. | desgl. | rot |

**Patentansprüche**

1. Wasserlösliche, symmetrische Disazoverbindungen der allgemeinen Formel (1)

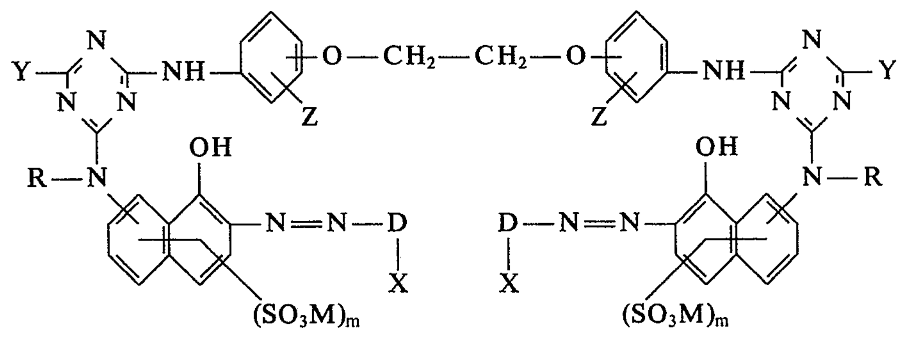

(1)

in welcher die zweifach auftretenden Formelglieder D, R, X, Y, Z, M und m jeweils eine einander identische Bedeutung besitzen und die Gruppen Z an die Benzolkerne jeweils in ortho- oder jeweils in para-Stellung zum Äthylendioxy-Substituenten sowie die Aminogruppen —NH— und die Gruppen Z in den Benzolkernen jeweils zueinander meta-ständig gebunden sind und

D der Rest einer aromatischen Disazokomponente ist,
X ein Wasserstoffatom oder eine faserreaktive Gruppe bedeutet, wobei X auch die Bedeutung des nachstehend definierten Formelrestes Z haben kann,
Y ein Fluoratom oder ein Chloratom ist,
Z eine Gruppe der Formel $-SO_2-CH=CH_2$ oder der Formel $-SO_2-CH_2-CH_2-R'$ bedeutet, in welcher
R' einen im wäßrigen Medium alkalisch eliminierbaren Rest darstellt oder für die Hydroxygruppe steht,
M ein Wasserstoffatom oder ein Alkalimetall oder das Äquivalent eines Erdalkalimetalls ist,
R ein Wasserstoffatom oder eine Alkylgruppe von 1 bis 4 C-Atomen bedeutet und
m die Zahl 1 oder 2 ist.

2. Disazoverbindungen nach Anspruch 1, in welchen hier D ein Phenylenrest ist, der durch Substituenten aus der Gruppe Halogen, Sulfo, Sulfamoyl, durch niederes Alkyl, Phenyl und/oder niederes Phenylalkyl mono- oder disubstituiertes Sulfamoyl, niederes Alkyl, niederes Alkoxy, Aryloxy, Carboxy, Carbamoyl, durch niederes Alkyl, Phenyl und/oder niederes Phenylalkyl mono- oder disubstituiertes Carbamoyl, niederes Alkyloxycarbonyl, niederes Alkanoylamino, niederes Arylalkanoylamino und gegebenenfalls durch niederes Alkyl substituiertes Aryloylamino substituiert sein kann.

3. Disazoverbindungen nach Anspruch 1, in welchen hier D ein Naphthylenrest ist, der durch eine, zwei oder drei Sulfogruppen und/oder durch ein Chloratom, eine niedere Alkylgruppe, eine niedere Alkoxygruppe, eine Carboxygruppe, eine niedere Alkanoylaminogruppe oder eine Benzoylaminogruppe substituiert sein kann.

4. Disazoverbindungen nach Anspruch 1, in welchen hier D in Phenylenrest ist, der durch einen oder zwei Substituenten aus der Gruppe Chlor, Brom, Sulfo, niederes Alkyl, niederes Alkoxy und Carboxy substituiert sein kann.

5. Disazoverbindungen nach Anspruch 1, in welchen hier D ein Naphthylenrest ist, der durch eine, zwei oder drei Sulfogruppen substituiert ist.

6. Disazoverbindungen nach einem der Ansprüche 1 bis 5, in welchen hier X jeweils für ein über eine Aminogruppe der Formel —NR″— an D gebundener 2,3-Dichlorchinoxalincarbonyl-, 2,4-Difluor-5-chlor-pyrimidyl-, 2,4-Dichlor-s-triazinyl-, 2-Chlor-4-(niederes Alkoxy)-s-triazinyl-, 2-Fluor-4-amino-s-triazinyl- oder 2-Chlor-4-amino-s-triazinyl-Rest oder für einen 2-Fluor- oder 2-Chlor-4-amino-s-triazinyl-Rest, in welchen jeweils die Aminogruppe durch niedere Alkyl-, Aryl- und/oder niedere Aralkyl-Gruppen substituiert sind, steht, wobei R″ ein Wasserstoffatom oder eine Alkylgruppe von 1 bis 4 C-Atomen bedeutet.

7. Disazoverbindungen nach einem der Ansprüche 1 bis 6, in welchen hier Z die Vinylsulfonylgruppe oder bevorzugt eine β-Sulfatoäthylsulfonyl-Gruppe bedeutet.

8. Disazoverbindungen nach einem der Ansprüche 1 bis 6, in welchen hier Z und X beide für die Vinylsulfonylgruppe oder bevorzugt eine β-Sulfatoäthylsulfonyl-Gruppe stehen.

9. Disazoverbindungen nach einem der Ansprüche 1 bis 7, in welchen hier X ein Wasserstoffatom bedeutet.

10. Verbindungen der allgemeinen Formel (2)

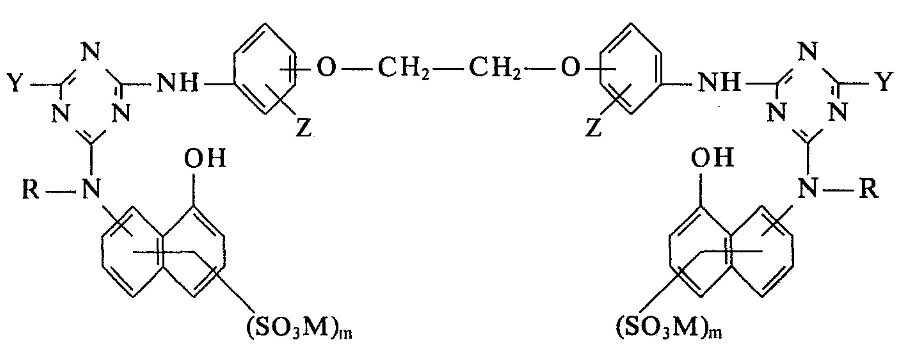

(2)

in welcher die zweifach auftretenden Formelglieder R, Y, Z, M und m jeweils eine einander identische Bedeutung besitzen und die Gruppen Z an die Benzolkerne jeweils in ortho- oder jeweils in para-Stellung zum Äthylendioxy-Substituenten sowei die Aminogruppen —NH— und die Gruppen Z in den Benzolkernen jeweils zueinander meta-ständig gebunden sind und

Y ein Fluoratom oder ein Chloratom ist,
Z eine Gruppe der Formel $-SO_2-CH=CH_2$ oder der Formel $-SO_2-CH_2-CH_2-R'$ bedeutet, in welcher
R′ einen im wäßrigen Medium alkalisch eliminierbaren Rest darstellt oder für die Hydroxygruppe steht,
M ein Wasserstoffatom oder ein Alkalimetall oder das Äquivalent eines Erdalkalimetalls ist,
R ein Wasserstoffatom oder eine Alkylgruppe von 1 bis 4 C-Atomen bedeutet und
m die Zahl 1 oder 2 ist.

11. Verbindungen nach Anspruch 10, in welchen Z hier eine β-Hydroxyäthylsulfonyl- oder die Vinylsulfonyl- oder eine β-Sulfatoäthylsulfonyl-Gruppe ist.

12. Verfahren zur Herstellung der Disazoverbindungen der allgemeinen Formel (1) von Anspruch 1, dadurch gekennzeichnet, daß man eine zweifach ankuppelbare Verbindung der allgemeinen Formel (2)

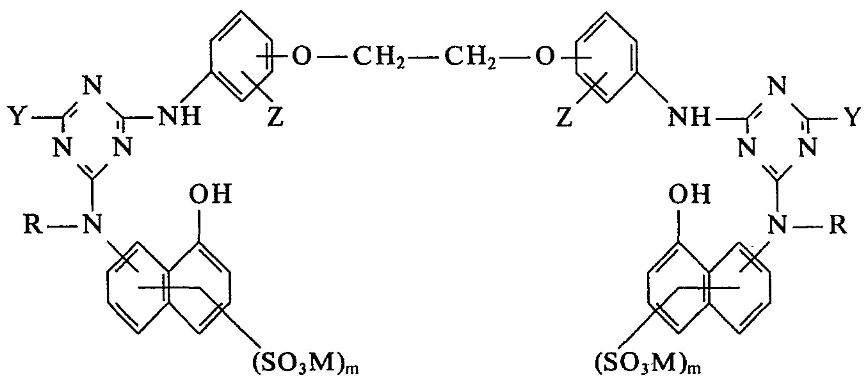

(2)

in welcher die zweifach auftretenden Formelglieder M, R, Y, Z und m jeweils eine einander identische, im Anspruch 1 genannte Bedeutung besitzen und die Aminogruppen —NH—, die Gruppen Z und die Äthylendioxy-Gruppe zueinander, wie in Anspruch 1 für Formel (1) angegeben, an die Benzolkerne gebunden sind, mit der äquivalenten Menge der Diazoniumverbindung eines aromatischen Amins der allgemeinen Formel (3)

$$H_2N—D—X \qquad (3)$$

in welcher D und X die in Anspruch 1 genannten Bedeutungen haben, kuppelt, oder daß man eine Azoverbindung der allgemeinen Formel (4)

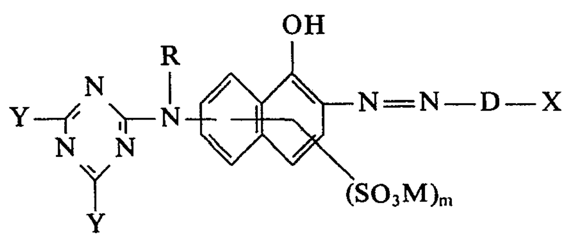

(4)

in welcher D, M, R, X, Y und m die in Anspruch 1 genannten Bedeutungen haben, in wäßrigem Medium bei einer Temperatur zwischen 20 und 50°C und bei einem pH-Wert zwischen 2,0 und 7,0 in äquivalenter Menge mit einer symmetrischen Diaminoverbindung der allgemeinen Formel (5)

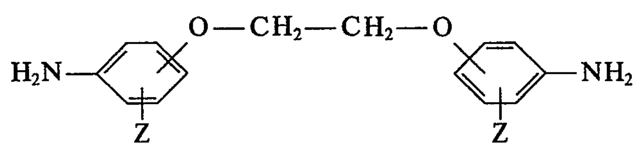

(5)

in welcher Z die in Anspruch 1 genannte, jeweils gleiche Bedeutung besitzt und die Gruppen Z an die Benzolkerne jeweils in ortho- oder jeweils in para-Stellung zum Äthylendioxy-Substituenten und die Aminogruppen und die Gruppen Z in den Benzolkernen jeweils zueinander meta-ständig gebunden sind, umsetzt.

13. Verwendung der Disazoverbindungen von Anspruch 1 oder der nach Anspruch 12 hergestellten Disazoverbindungen als Farbstoffe.

14. Verwendung nach Anspruch 13 zum Färben oder Bedrucken von hydroxy-, amino- und/oder carbonamidgruppenhaltigen Fasermaterialien.

15. Verfahren zur Herstellung der Verbindungen der allgemeinen Formel (2) von Anspruch 10, dadurch gekennzeichnet, daß man eine Verbindung der allgemeinen Formel (6)

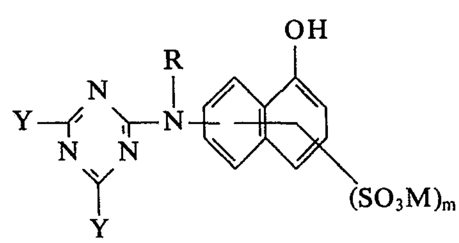

(6)

in welcher R, Y, M und m die in Anspruch 10 genannten Bedeutungen haben, mit der äquivalenten Menge eines Diamins der allgemeinen Formel (5)

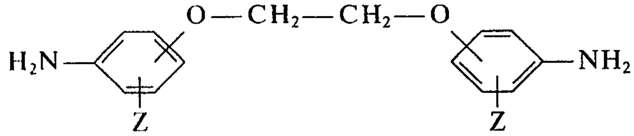
(5)

in welcher Z die in Anspruch 10 genannte, jeweils gleiche Bedeutung besitzt und die Gruppen Z an die Benzolkerne jeweils in ortho- oder jeweils in para-Stellung zum Äthylendioxy-Substituenten und die Aminogruppen und die Gruppen Z in den Benzolkernen jeweils zueinander meta-ständig gebunden sind, bei einer Temperatur zwischen 5 und 50°C und einem pH-Wert zwischen 3,5 und 7,0 umsetzt oder daß man eine Diaminoverbindung der obengenannten und definierten allgemeinen Formel (5) mit der zweifach molaren Menge an 2,4,6-Trifluor-s-triazin oder 2,4,6-Trichlor-s-triazin in wäßrigem oder wäßrig-organischem Medium bei einer Temperatur zwischen −10°C und +15°C und einem pH-Wert zwischen 2,5 und 5,5 zur symmetrischen Verbindung der allgemeinen Formel (7)

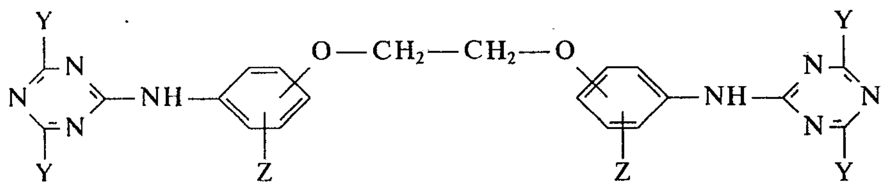
(7)

in welcher jedes Y und jedes Z die gleiche, obengenannte Bedeutung besitzt und die Äthylendioxygruppe, die Gruppen −NH− und die Gruppen Z zueinander, wie in Anspruch 10 für Formel (2) angegeben, an die Benzolkerne gebunden sind, umsetzt und diese Verbindung (7) sodann mit einer Aminonaphtholsulfonsäure der allgemeinen Formel (8)

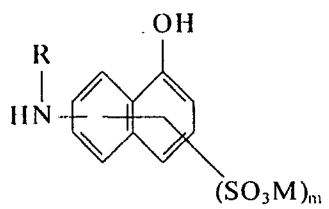
(8)

in welcher M, R und m die obengenannten Bedeutungen haben, in äquivalenter Menge kondensiert.

16. Verwendung der Verbindungen der allgemeinen Formel (2) von Anspruch 10 oder der nach Anspruch 15 hergestellten Verbindungen der allgemeinen Formel (2) als Kupplungskomponenten zur Herstellung von Azoverbindungen.

## Claims

1. Water-soluble, symmetrical disazo compounds of the general formula (1)

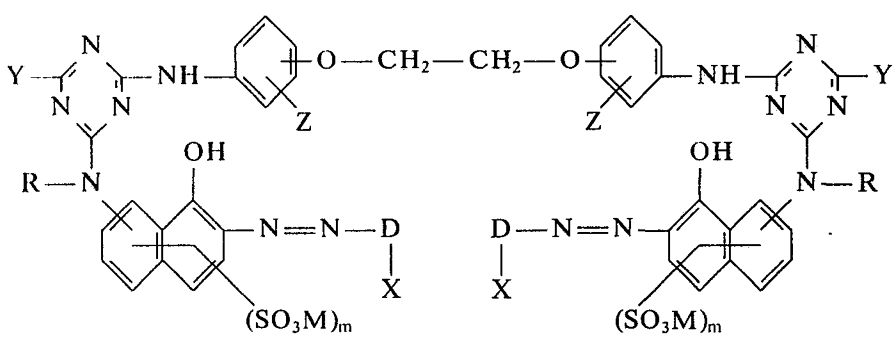
(1)

in which the formula moieties D, R, X, Y, Z, M and m, occurring twice, are in each case identical to each other and the groups Z are bonded to the benzene nuclei in each case in ortho- or in each case in para-position relative to the ethylenedioxy substituent, and the amino groups −NH− and the groups Z are bonded in the benzene nuclei in each case in meta-position relative to each other and

D is the radical of an aromatic diazo component,
X denotes a hydrogen atom or a fiber-reactive group which can also have the meaning of the formula member Z defined below,
Y is a fluorine atom or a chlorine atom,
Z denotes a group of the formula $-SO_2-CH=CH_2$ or $-SO_2-CH_2-CH_2-R'$ in which
R' represents a radical which can be eliminated in an aqueous medium under alkaline conditions, or the hydroxy group,
M is a hydrogen atom or an alkali metal or the equivalent of an alkaline earth metal,
R denotes a hydrogen atom or an alkyl group of 1 to 4 C-atoms and
m is the number 1 or 2.

2. Disazo compounds according to claim 1, in which D is a phenylene radical which can be substituted by substituents from the group consisting of halogen, sulfo, sulfamoyl, sulfamoyl monosubstituted or disubstituted by lower alkyl, phenyl and/or lower phenylalkyl, lower alkyl, lower alkoxy, aryloxy, carboxy, carbamoyl, carbamoyl monosubstituted or disubstituted by lower alkyl, phenyl and/or lower phenylalkyl, lower alkyloxycarbonyl, lower alkanoylamino, lower arylalkanoylamino and aryloylamino optionally substituted by lower alkyl.

3. Disazo compounds according to claim 1, in which D is a naphthylene radical which can be substituted by one, two or three sulfo groups and/or by one chlorine atom, 1 lower alkyl group, 1 lower alkoxy group, 1 carboxy group, 1 lower alkanoylamino group or 1 benzoylamino group.

4. Disazo compounds according to claim 1, in which D is a phenylene radical which can be substituted by one or two substituents from the group consisting of chlorine, bromine, sulfo, lower alkyl, lower alkoxy and carboxy.

5. Disazo compounds according to claim 1, in which D is a naphthylene radical which is substituted by one, two or three sulfo groups.

6. Disazo compounds according to any of claims 1 to 5, in which the X are bonded to D via an amino group of the formula $-NR''-$ in which R" denotes a hydrogen atom or an alkyl group of 1 to 4 C-atoms, and both represent each a 2,3-dichloroquinoxalinecarbonyl, 2,4-difluoro-5-chloropyrimidyl, 2,4-dichloro-s-triazinyl, 2-chloro-4-(lower alkoxy)-s-triazinyl, 2-fluoro-4-amino-s-triazinyl or 2-chloro-4-amino-s-triazinyl group or a 2-fluoro- or 2-chloro-4-amino-s-triazinyl group, the amino groups of which are substituted by lower alkyl, aryl and/or lower aralkyl groups.

7. Disazo compounds according to any of claims 1 to 6, in which Z denotes the vinylsulfonyl group or, preferably, a β-sulfatoethylsulfonyl group.

8. Disazo compounds according to any of claims 1 to 6, in which Z and X both represent the vinylsulfonyl group or, preferably, a β-sulfatoethylsulfonyl group.

9. Disazo compounds according to any of claims 1 to 7, in which X denotes a hydrogen atom.

10. Compounds of the general formula (2)

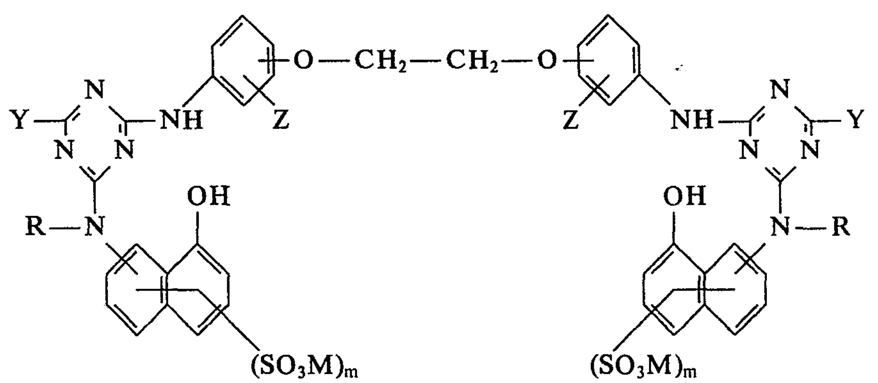

(2)

in which the formula moieties R, Y, Z, M and m, occurring twice, are in each case identical to each other and the groups Z are bonded to the benzene nuclei in each case in ortho- or in each case in para-position relative to the ethylenedioxy substituent, and the amino groups $-NH-$ and the groups Z are bonded in the benzene nuclei in each case in meta-position relative to each other and

Y is a fluorine atom or a chlorine atom,
Z denotes a group of the formula $-SO_2-CH=CH_2$ or $-SO_2-CH_2-CH_2-R'$ in which
R' represents a radical which can be eliminated in an aqueous medium under alkaline conditions, or the hydroxy group,
M is a hydrogen atom or an alkali metal or the equivalent of an alkaline earth metal,
R denotes a hydrogen atom or an alkyl group of 1 to 4 C-atoms and
m is the number 1 or 2.

11. Compounds according to claim 10, in which Z is a $\beta$-hydroxyethylsulfonyl or the vinylsulfonyl or a $\beta$-sulfatoethylsulfonyl group.

12. A process for the preparation of the disazo compounds of the general formula (1), of claim 1, characterized by that a doubly couplable compound of the general formula (2)

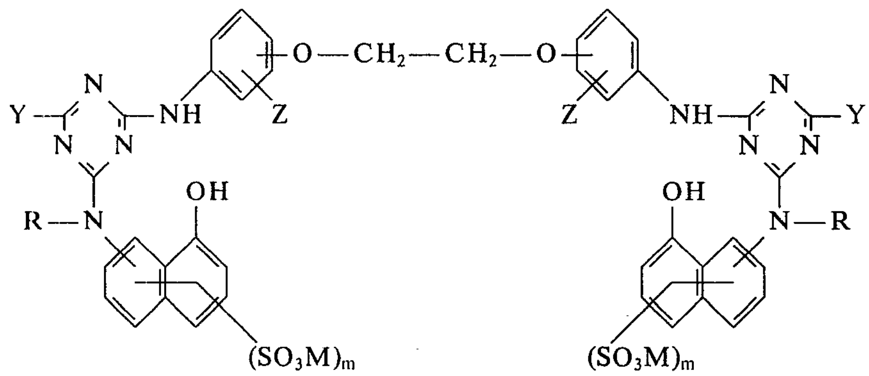

(2)

in which the formula moieties M, R, Y, Z and m, occurring twice, in each case have a meaning identical to each other and as mentioned in claim 1, and the amino groups —NH—, the groups Z and the ethylenedioxy group are bonded to the benzene nuclei relative to one another as indicated in claim 1 for the formula (1), is coupled with the equivalent amount of the diazonium compound of an aromatic amine of the general formula (3)

$$H_2N—D—X \qquad (3)$$

in which D and X have the meanings mentioned in claim 1, or that an azo compound of the general formula (4)

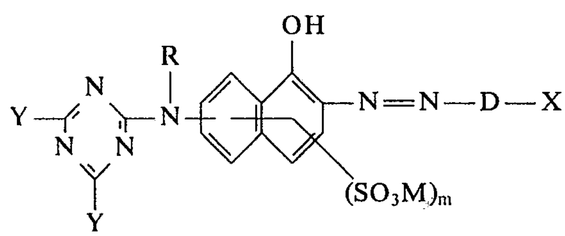

(4)

in which D, M, R, X, Y and m have the meanings mentioned in claim 1, is reacted in an aqueous medium at a temperature between 20 and 50°C and at a pH value between 2.0 and 7.0 in an equivalent amount with a symmetrical diamino compound of the general formula (5)

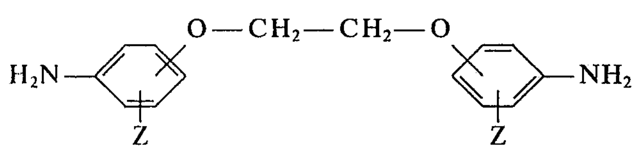

(5)

in which both Z have the same meaning as mentioned in claim 1, and the groups Z are bonded to the benzene nuclei in each case in ortho- or in each case in para-position relative to the ethylenedioxy substituent, and the amino groups and the groups Z are bonded in the benzene nuclei in each case in meta-position relative to each other.

13. The use of the disazo compounds of claim 1 or of the disazo compounds prepared according to claim 12, as dyestuffs.

14. The use according to claim 13, for dyeing or printing fiber materials containing hydroxy, amino and/or carboxamide groups.

15. A process for the preparation of compounds of the general formula (2) of claim 10, characterized by that a compound of the general formula (6)

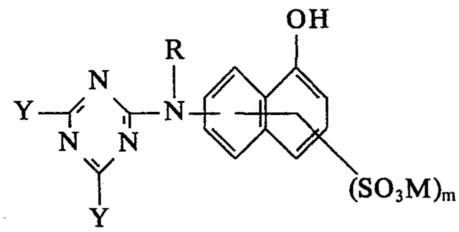

(6)

in which R, Y, M and m have the meanings mentioned in claim 10, is reacted with the equivalent amount of a diamine of the general formula (5)

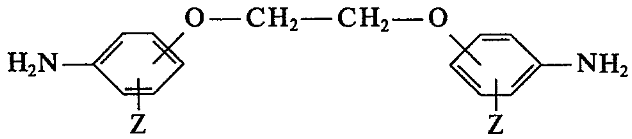

(5)

in which both Z have the same meaning as mentioned in claim 10, and the groups Z are bonded to the benzene nuclei in each case in ortho- or in each case in para-position relative to the ethylenedioxy substituent, and the amigo groups and the groups Z are bonded in the benzene nuclei in each case in meta-position relative to each other, at a temperature between 5 and 50°C and at a pH value between 3.5 and 7.0, or that a diamino compound of the above-mentioned and defined formula (5) is reacted with the two-fold molar amount of 2,4,6-trifluoro-s-triazine or 2,4,6-trichloro-s-triazine in an aqueous or aqueous-organic medium at a temperature between −10°C and +15°C and at a pH value between 2.5 and 5.5 to give the symmetrical compound of the general formula (7)

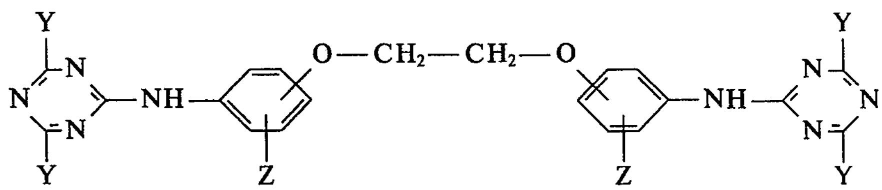

(7)

in which each Y and each Z has the same, abovementioned meaning and the ethylenedioxy group, the groups −NH− and the groups Z are bonded to the benzene nuclei relative to one another as indicated in claim 10 for the formula (2), and this compound (7) is then condensed with an aminonaphtholsulfonic acid of the general formula (8)

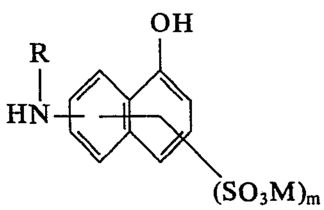

(8)

in which M, R and m have the abovementioned meanings, in equivalent amount.

16. The use of the compounds of the general formula (2) of claim 10 or of the compounds prepared according to claim 15, of the formula (2), as coupling components for preparing azo compounds.

**Revendications**

1. Composés disazoïques symétriques, solubles dans l'eau, qui répondent à la formule générale 1:

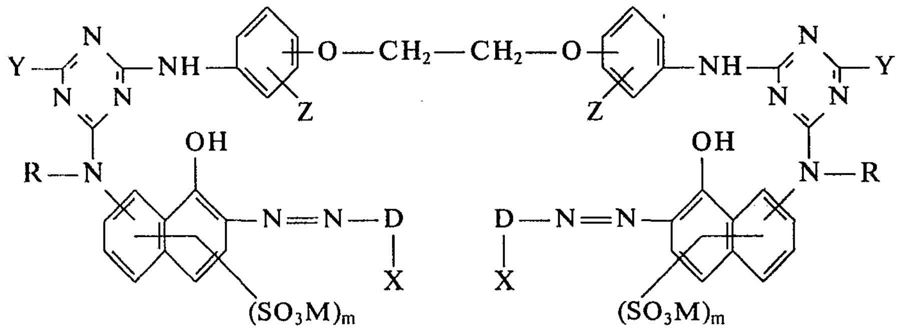

(1)

dans laquelle les deux éléments de chacun des couples de radicaux apparaissant deux fois, soit D, R, X, Y, Z, M et m, sont identiques l'un à l'autre, les radicaux Z portés par des noyaux benzéniques sont chacun en position ortho ou chacun en position para relativement au substituant éthylène-dioxy, et les radicaux amino — NH — et Z se trouvent, sur chacun des noyaux benzéniques, en position méta l'un par rapport à l'autre,

D représente le radical d'une composantede diazotation aromatique,
X représente un atome d'hydrogène ou un radical réactif à l'égard des fibres et peut également avoir la signification du radical Z indiquée ci-dessous,
Y représente un atome de fluor ou de chlore,
Z représente un radical $-SO_2-CH=CH_2$ ou un radical $-SO_2-CH_2-CH_2-R'$ dans lequel
R' désigne un radical pouvant être éliminé en milieu alcalin aqueux ou un radical hydroxy,
M représente un atome d'hyrogène, un métal alcalin ou un équivalent d'un métal alcalinoterreux,
R représente un atome d'hydrogène ou un radical alkyle contenant de 1 à 4 atomes de carbone et
m représente le nombre 1 ou le nombre 2.

2. Composés disazoïques selon la revendication 1, dans lesquels D représente un radical phénylène qui peut porter des substituants pris dans l'ensemble constitué par les halogènes, le sulfo, le sulfamoyle, les sulfamoyles monoou disubstitués (les substituants étant alors pris dans l'ensemble constitué par les alkyles inférieurs, le phényle et les phénylalkyles inférieurs), les alkyles inférieurs, les alcoxy inférieurs, les aryloxy, le carboxy, le carbamoyle, les carbamoyles mono ou disubstitués (les substituants seront pris dans l'ensemble constitué par les alkyles inférieurs, le phényle et les phénylalkyles inférieurs), est alcoxy-carbonyles inférieurs, les alcanoylamino inférieurs, les arylalcanoylamino inférieurs et les aroylamino éventuellement porteurs d'alkyles inférieurs.

3. Composés disazoïques selon la revendication 1, dans lesquels D représente un radical naphthylène qui peut porter un, deux ou trois radicaux sulfo et/ou un atome de chlore, un radical alkyle inférieur, un radical alcoxy inférieur, un radical carboxy, un radical alcanoylamino inférieur ou un radical benzoyl-amino.

4. Composés disazoïques selon la revendication 1, dans lesquels D représente un radical phénylène qui peut porter un ou deux substituants pris dans l'ensemble constitué par le chlore, le brome, le sulfo, les alkyles inférieurs, les alcoxy inférieurs et le carboxy.

5. Composés disazoïques selon la revendication 1, dans lesquels D représente un radical naphtylène portant un, deux ou trois radicaux sulfo.

6. Composés disazoïques selon l'une quelconque des revendications 1 à 5, dans lesquels les symboles X représentent chacun un radical dichloro-2,3 quinoxaline-carbonyle, difluoro-2,4 chloro-5 pyrimidyle, dichloro-2,4 s-triazinyle, chloro-2 (alcoxy inférieur)-4 s-triazinyle, fluoro-2 amino-4 s-triazinyle ou chloro-2 amino-4 s-triazinyle, relié à D par un radical amino de formule — NR″ —, ou un radical fluoro-2 ou chloro-2 amino-4 s-triazinyle, le radical amino pouvant porter à chaque fois un alkyle inférieur, un aryle et/ou un aralkyle inférieur et R″ représentant un atome d'hydrogène ou un alkyle contenant de un à quatre atomes de carbone.

7. Composés disazoïques selon l'une quelconque des revendications 1 à 6, dans lesquels Z représente un radical vinylsulfonyle ou, mieux, un radical sulfato-2- éthylsulfonyle.

8. Composés disazoïques selon l'une quelconque des revendications 1 à 6, dans lesquels Z et X représentent chacun un radical vinylsulfonyle ou, mieux, un radical sulfato-2 éthylsulfonyle.

9. Composés disazoïques selon l'une quelconque des revendications 1 à 7, dans lesquels X représente un atome d'hydrogène.

10. Composés répondant à la formule générale 2:

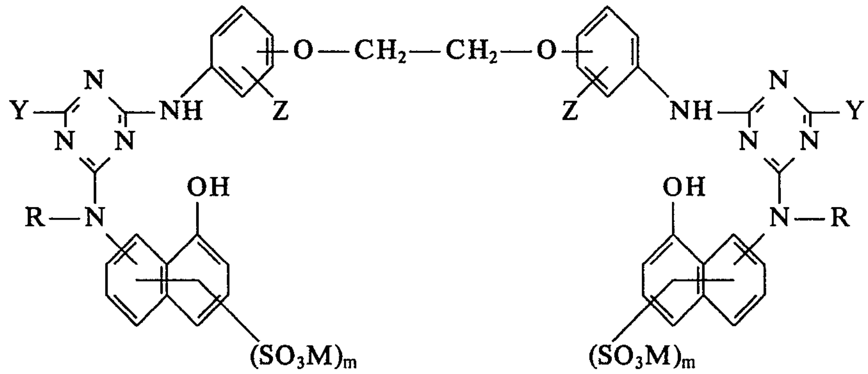

(2)

dans laquelle les deux éléments de chacun des couples de radicaux qui apparaissent deux fois, soit R, Y, Z, M et m, sont identiques l'un à l'autre, les radicaux Z portés par les noyaux benzéniques sont chacun en position ortho ou chacun en position para relativement au substituant éthylène-dioxy, et les radicaux amino $-NH-$ et Z se trouvent, sur chacun des noyaux benzéniques, en position méta l'un par rapport à l'autre,

Y représente un atome de fluor ou de chlore,
Z représente un radical $-SO_2-CH=CH_2$ ou un radical $-SO_2-CH_2-CH_2-R'$ dans lequel
R' désigne un radical pouvant être éliminé en milieu alcalin aqueux ou un radical hydroxy,
M représente un atome d'hydrogène ou un métal alcalin ou l'équivalent d'un métal alcalino-terreux,
R représente un atome d'hydrogène ou un radical alkyle contenant de 1 à 4 atomes de carbone et
m représente le nombre 1 ou le nombre 2.

11. Composés selon la revendication 10, dans lesquels Z représente un radical hydroxy-2 éthylsulfonyle, un radical vinylsulfonyle ou un radical sulfato-2 éthylsulfonyle.

12. Procédé de préparation de composés disazoïques de formule générale 1 selon la revendication 1, procédé caractérisé en ce qu'on copule un composé bicopulable répondant à la formule générale 2:

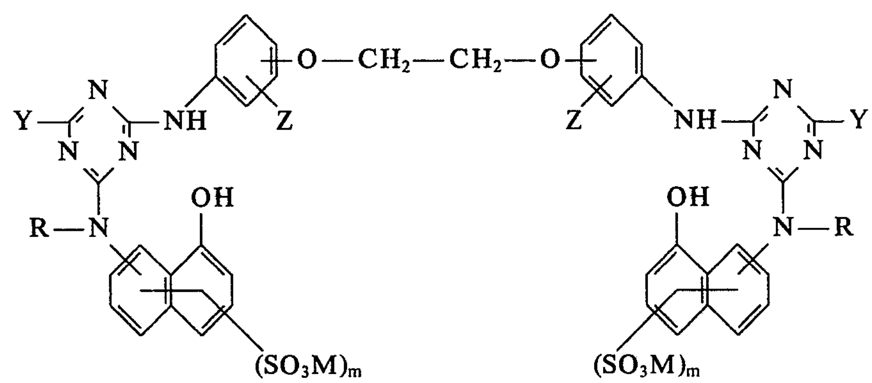

(2)

dans laquelle les symboles identiques figurant deux fois, c'est c'est-à-dire M, R, Y, Z et m, représentent des radicaux identiques ayant les significations données à la revendication 1, et dans laquelle les radicaux amino $-NH-$, les radicaux Z et le radical éthylène-dioxy sont disposés les uns par rapport aux autres, sur les noyaux benzéniques, comme indiqué à la revendication 1 à propos de la formule 1, avec la quantité équivalente du composé de diazonium d'une amine aromatique répondant à la formule générale 3:

$$H_2N-D-X \qquad (3)$$

dans laquelle D et X ont les significations données à la revendication 1, ou on fait réagir un composé azoïque répondant à la formule générale 4:

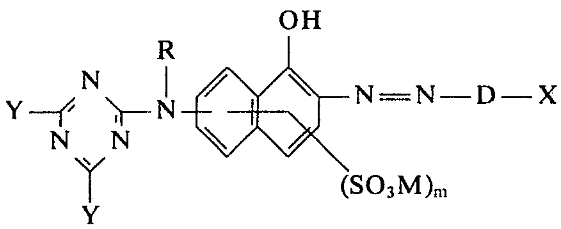

(4)

dans laquelle D, M, R, X, Y et m ont les significations données à la revendication 1, en milieu aqueux, à une température comprise entre 20 et 50°C et à un pH compris entre 2,0 et 7,0, en des quantités équivalentes, avec un composé diaminé symétrique répondant à la formule générale 5:

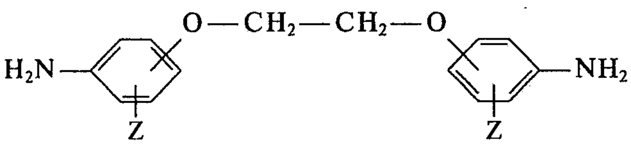

(5)

dans laquelle les Z sont identiques l'un à l'autre et ont les significations données à la revendication 1 et, sur chacun des noyaux benzéniques, Z est en ortho ou en para relativement au radical éthylène-dioxy tandis que le radical amino et Z sont en méta l'un par rapport à l'autre.

13. Application de composés disazoïques selon la revendication 1, ou des composés disazoïques préparés selon la revendication 12, comme colorants.

14. Application selon la revendication 13 pour la teinture ou l'impression de matières fibreuses contenant des radicaux hydroxy, des radicaux amino et/ou des radicaux carbamoyles.

15. Procédé de préparation des composés de formule générale 2 selon la revendication 10, procédé caractérisé en ce qu'on fait reagir un composé répondant à la formule générale 6:

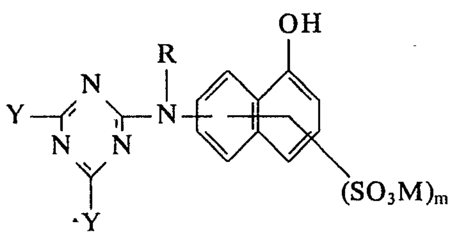

(6)

dans laquelle R, Y, M et m ont les significations données à la revendication 10, avec la quantité équivalente d'une diamine répondant à la formule générale 5:

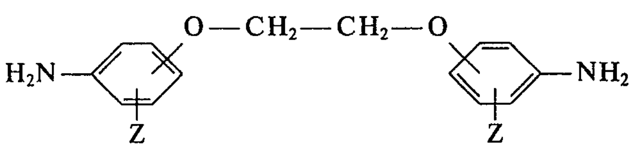

(5)

dans laquelle les Z représentent des radicaux identiques et ont les significations qui ont été données à la revendication 10 et, sur chacun des noyaux benzéniques, Z est en position ortho ou en position para relativement au radical éthylènedioxy, tandis que le radical amino et le radical Z sont, l'un par rapport à l'autre, en position méta, à une température comprise entre 5 et 50°C et à un pH compris entre 3,5 et 7,0, ou on fait réagir un composé diaminé répondant à la formule générale 5 qui vient d'être représentée et définie, avec le double de la quantité molaire de trifluoro-2,4,6 triazine-1,3,5 ou de trichloro-2,4,6 triazine-1,3,5, en milieu aqueux ou aqueux-organique, à une température comprise entre −10 et +15°C et à un pH compris entre 2,5 et 5,5, réaction qui conduit au composé symétrique répondant à la formule générale 7:

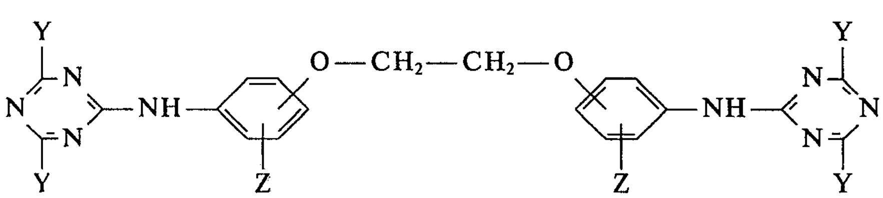

(7)

dans laquelle les Y, de même que les Z, représentent des radicaux identiques et ont les significations

indiquées cidessus, le radical éthylène-dioxy, le radical — NH — et le radical Z sont disposés, sur chacun des noyaux benzéniques, comme cela a été dit à la revendication 10 à propos de la formule 2, puis on condense ce composé (7) avec un acide amino-hydroxy-naphtalène-sulfonique répondant à la formule générale 8:

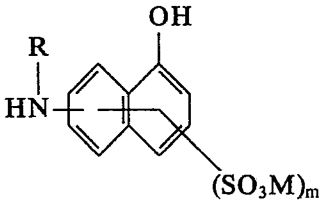

(8)

dans laquelle, M, R et m ont les significations précédemment données, en des quantités équivalentes.

16. Application des composés de formule générale 2 selon la revendication 10, ou des composés de formule générale 2 préparés selon la revendication 15, comme copulants pour la préparation de composés azoïques.